(19) Europäisches Patentamt
European Patent Office
Office européen des brevets

(11) EP 1 296 946 B1

(12) # FASCICULE DE BREVET EUROPEEN

(45) Date de publication et mention de la délivrance du brevet:
**21.04.2004 Bulletin 2004/17**

(21) Numéro de dépôt: **01940654.5**

(22) Date de dépôt: **31.05.2001**

(51) Int Cl.[7]: **C07C 327/22**, C07D 295/14, A61K 31/255, A61K 31/40, A61K 31/535

(86) Numéro de dépôt international:
**PCT/FR2001/001694**

(87) Numéro de publication internationale:
**WO 2001/092220 (06.12.2001 Gazette 2001/49)**

(54) **COMPOSES AMINOTHIOLESTERS, COMPOSITIONS PHARMACEUTIQUES ET COSMETIQUES LES CONTENANT ET UTILISATIONS**

AMINO-THIOLESTER-VERBINDUNGEN, DIESE ENTHALTENDE PHARMAZEUTISCHE UND KOSMETISCHE ZUSAMMENSETZUNGEN UND IHRE ANWENDUNGEN

AMINOTHIOLESTER COMPOUNDS, PHARMACEUTICAL AND COSMETIC COMPOSITIONS CONTAINING SAME AND USES THEREOF

(84) Etats contractants désignés:
**AT BE CH CY DE DK ES FI FR GB GR IE IT LI LU MC NL PT SE TR**

(30) Priorité: **31.05.2000 FR 0007029**

(43) Date de publication de la demande:
**02.04.2003 Bulletin 2003/14**

(73) Titulaire: **Galderma Research & Development, S.N.C.**
**06560 Valbonne (FR)**

(72) Inventeurs:
- **FOURNET, Guy**
  **F-69100 Villeurbanne (FR)**
- **QUASH, Gérard, Antony**
  **F-69340 Francheville (FR)**
- **GORE, Jacques**
  **F-69300 Caluire et Cuire (FR)**

(74) Mandataire: **Allab, Myriam**
**L'OREAL - D.I.P.I.**
**25-29 Quai Aulagnier**
**92600 Asnières (FR)**

(56) Documents cités:
**EP-A- 0 947 503** **WO-A-98/44919**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des composés aminothiolesters de formule (I) ainsi que leur utilisation en tant qu'agents inducteurs de l'apoptose cellulaire. Les composés selon l'invention ont une activité marquée comme agent inducteur d'apoptose et trouvent des applications plus particulièrement dans le traitement topique et systémique des cancers et précancers, des affections dermatologiques, rhumatismales et ophtalmologiques.
La présente invention a également pour objet une composition pharmaceutique ou cosmétique contenant, en tant qu'agent actif, au moins un composé aminothiolester de formule (I), dans un excipient physiologiquement acceptable.
Par le terme "apoptose", on entend le phénomène de mort cellulaire tel que décrit entre autres par KERR J. F.R. et al., J. Cancer, 265, 239 (1972). L'apoptose qui est une forme hautement sélective de suicide cellulaire, se caractérise par des phénomènes morphologiques et biochimiques aisément observables. Ainsi, on observe une condensation de la chromatine associée ou non à une activité endonucléasique, la formation de corps apoptotiques et une fragmentation de l'acide désoxyribonucléique (ADN) du fait de l'activation d'endonucléases, en fragments d'ADN de 180-200 paires de base donnant un profil facilement reconnaissable par électrophorèse sur gel d'agarose.
L'apoptose est impliquée dans le développement, la différentiation et le renouvellement tissulaire. L'induction d'apoptose présente donc un grand intérêt d'un point de vue thérapeutique, mais également d'un point de vue cosmétique.
Une très grande variété de médicaments anticancéreux naturels ou synthétiques actuellement disponibles sont des composés inducteurs d'apoptose.
Parmi ces médicaments antinéoplasiques, on peut citer les agents alkylants tels que la cyclophosphamide, les nitrosourées tels que le 1,3-bis (2-chloroéthyl)-1-nitrosourée (BCNU), les agents intercalants tels que l'actinomycine D ou l'adriamycine, les analogues de bases puriques ou pyrimidiques tels que la 6-thioguanine et la 5-fluorouracile, les inhibiteurs de la synthèse de novo des bases puriques tels que le méthotrexate et enfin les inhibiteurs de la polymérisation de la tubuline tels que le Taxol®.
Par ailleurs, il a déjà été proposé par la demanderesse, notamment dans la demande de brevet WO96/20701, d'utiliser un composé inducteur d'apoptose choisi parmi le méthional, le malonaldéhyde et tout facteur permettant d'augmenter le taux intracellulaire de ces composés, c'est-à-dire ayant une action sur le métabolisme du méthional représenté pour mémoire à la **Figure 1** (QUASH et al., Biochem. J. 305, 1017 (1995)).
Il a également été décrit dans cette même demande de brevet, à titre de facteur augmentant le taux intracellulaire du méthional, des inhibiteurs de l'acide 4-méthylthio 2-oxobutanoique (MTOB) transaminase, composés d'esters de L-méthionine et de pyridoxal. La MTOB transaminase étant une enzyme impliquée dans la conversion de l'acide 4-méthylthio 2-oxobutanoïque en méthionine, l'utilisation de ces composés favorise l'accumulation de MTOB, précurseur direct du méthional (Figure 1) (ROCH et al., Biochem. J. 313, 973 (1996)).
Un des principaux inconvénients de l'utilisation de ces substances est l'absence d'une activité apoptotique sélective des cellules tumorales. Ainsi, il reste nécessaire de disposer de molécules qui induisent une apoptose maximale dans le tissu tumoral tout en lésant le moins possible et de façon réversible les tissus sains de l'organisme.
En vue de remédier à cette absence de sélectivité, la demanderesse a décrit dans la demande de brevet WO-98/44919, l'utilisation de dérivés aminothiolesters, dont le thioampal, en tant qu'inhibiteurs de l'enzyme aldéhyde déshydrogénase (ALDH), enzyme impliquée dans la conversion du méthional en acide méthylthiopropionique, favorisant ainsi l'accumulation de méthional. Ces composés sont des inhibiteurs sélectifs de la croissance des cellules transformées résistantes à l'apoptose du fait de la surexpression du gène $bcl_2$.
De tels composés sont donc plus spécifiquement destinés à traiter les pathologies caractérisées par une surexpression du gène $bcl_2$ telles que les cancers du sein, les lymphomes des cellules B, les leucémies, les neuroblastomes, les adénocarcinomes de la prostate, les prolactinomas et autres adénomes pituitaires.
Cependant ces dérivés aminothiolesters sont délicats à préparer. En effet, leurs procédés de préparation présentent un rendement ne dépassant pas 20%. De plus ces composés présentent des problèmes de stabilité et doivent être conservés à des températures d'environ -20°C pour éviter leur dégradation.
Il était donc souhaitable de mettre au point de nouveaux composés qui soient stables et aisés à préparer avec de bons rendements et permettant d'inhiber de façon sélective la croissance des cellules transformées résistantes à l'apoptose du fait de la surexpression du gène $bcl_2$.
C'est l'objet de la présente invention qui concerne de nouveaux composés aminothiolesters qui peuvent être représentés par la formule générale (**I**) suivante :

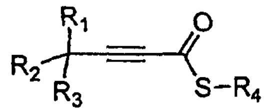

**(I)**

dans laquelle :

- $R_1$ représente:

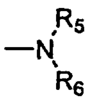 ou 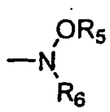 ou 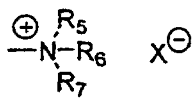 ou 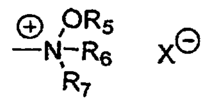

- $R_2$ et $R_3$, indépendamment, représentent un radical alkyle ayant de 1 à 7 atomes de carbone, saturé ou insaturé, cyclique, linéaire ou ramifié ou un radical aryle ayant de 1 à 7 atomes de carbone, ou un radical aralkyle ayant de 1 à 12 atomes de carbone ou pris ensemble forment un cycle alkyle saturé ayant de 3 à 7 atomes de carbone,
- $R_4$ représente un radical alkyle ayant de 1 à 7 atomes de carbone saturé ou insaturé, cyclique, linéaire ou ramifié ou un radical aryle ayant de 1 à 7 atomes de carbone ou un radical aralkyle ayant de 1 à 12 atomes de carbone,
- $R_5$ et $R_6$, indépendamment, représentent un radical alkyle ayant de 1 à 7 atomes de carbone saturé ou insaturé, cyclique, linéaire ou ramifié ou un radical aryle ayant de 1 à 7 atomes de carbone, ou un radical aralkyle ayant de 1 à 12 atomes de carbone ou pris ensemble forment avec l'atome d'azote un hétérocycle azoté saturé ou insaturé ayant de 2 à 6 atomes de carbone, éventuellement substitué par un oxygène, un soufre ou par un atome d'azote éventuellement substitué par un radical alkyle ayant de 1 à 7 atomes de carbone saturé ou insaturé, cyclique linéaire ou ramifié ou un radical aryle ayant de 1 à 7 atomes de carbone ou un radical aralkyle ayant de 1 à 12 atomes de carbone,
- $R_7$ représente un atome d'hydrogène, un radical alkyle ayant de 1 à 7 atomes de carbone saturé ou insaturé cyclique, linéaire ou ramifié ou un radical aryle ayant de 1 à 7 atomes de carbone ou un radical aralkyle ayant de 1 à 12 atomes de carbone, et
- X représente un ion halogénure, ou un anion nitrate, sulfate, sulfonate, carboxylate, thiocyanate, ou phosphate.

Parmi les radicaux alkyles cycliques ayant de 3 à 7 atomes de carbone, on peut citer notamment les radicaux cyclopropyle, cyclopentyle, cyclohexyle ou cycloheptyle. Parmi les radicaux alkyles saturés linéaires ayant de 1 à 7 atomes de carbone, on peut citer notamment les radicaux méthyle; éthyle, *n*-propyle, *n*-butyle, *n*-pentyle, *n*-hexyle ou *n*-heptyle.
Parmi les radicaux alkyles saturés et ramifiés ayant de 1 à 7 atomes de carbone, on peut citer notamment les radicaux *i*-propyle, *t*-butyle, 2-butyle, 2-pentyle, *i*-pentyle, 2-hexyle, 3-hexyle, 2-heptyle, 3-heptyle ou *i*-heptyle.
Parmi les radicaux alkyles insaturés ayant de 1 à 7 atomes de carbone, on peut citer notamment le radical allyle ou vinyle.
Par radical aralkyle ayant de 1 à 12 atomes de carbone, on entend ou un radical phényle éventuellement substitué par des radicaux alkyles linéaires ou ramifiés ayant de 1 à 5 atomes de carbone lié à une chaîne alkyle linéaire saturée ayant de 1 à 5 atomes de carbone tels que le radical benzyle éventuellement substitué par un radical alkyle ayant de 1 à 5 atomes de carbone, les radicaux 1-phényl éthyle, 1-phényle propyle, 1-phényle butyle, 1-phényle pentyle.
Parmi les radicaux aryles ayant de 1 à 7 atomes de carbone, on peut citer notamment les radicaux phényle, tolyle, chlorophényle, nitrophényle, méthoxyphényle, thiophène ou furane. Parmi les hétérocycles azotés on peut notamment citer la pyrrolidine, la pipéridine, la morpholine, la thiamorpholine, la pipérazine, l'homopipérazine ou la N-méthyl pipérazine. Parmi les halogénures on peut citer les fluorures, chlorures, bromures et en particulier les iodures.
Parmi les composés de formule (**I**) entrant dans le cadre de la présente invention, on peut notamment citer les suivants :

| N° | Composés |
|---|---|
| 1- | 4-Diméthylamino-4-méthyle-pent-2-ynethioate de S-méthyle |
| 2- | Iodure de 4-méthyle-4-triméthylammonium-pent-2-ynethioate de S-méthyle |

(suite)

| N° | Composés |
|---|---|
| 3- | 4-Di-*n*-propylamino-4-méthyle-pent-2-ynethioate de S-méthyle |
| 4- | 4-Méthyl-4-pyrrodin-1-yl-pent-2-ynethioate de S-méthyle |
| 5- | 4-Méthyl-4-morpholin-4-yl-pent-2-ynethioate de S-méthyle |

Selon la présente invention, les composés de formule (**I**) plus particulièrement préférés sont ceux pour lesquels l'une au moins des conditions ci-dessous est respectée :

- $R_2$ et $R_3$, représentent un radical méthyle ;
- $R_4$ représente un radical méthyle ;
- $R_5$ et $R_6$, indépendamment, représentent un radical alkyle ayant de 1 à 3 atomes de carbone ou pris ensemble forment avec l'atome d'azote un hétérocycle azoté choisi parmi la pyrrolidine, la pipéridine ou la morpholine ;
- $R_7$ représente un radical méthyle ou un atome d'hydrogène ;
- et X représente un ion iodure, un ion formiate ou un ion carboxylate.

La présente invention a également pour objet les procédés de préparation des composés de formule (I).

Les composés de formule (**I**) ($R_1 = N(R_5R_6)$) selon l'invention peuvent être obtenus selon le schéma réactionnel présenté sur la figure 2. Le procédé de synthèse consiste à faire réagir le butyllithium sur une amine propargylique de formule (**III**) dans du THF. Le lithien acétylénique intermédiairement formé est mis en réaction avec de l'oxysulfure de carbone (COS) puis avec l'iodure $R_4I$. Le thiolester de formule (I) ($R_1 = N(R_5R_6)$) est ainsi obtenu avec un rendement voisin de 70%.

On peut également envisager, comme cela est présenté sur la figure 3, la réaction du lithien acétylénique directement sur un dialkyl ou diaryl dithiocarbonate de formule (**IV**). Ces composés sont abondamment décrits, par exemple lorsque $R_4$ représente un radical méthyle [DOUGLASS, I.B., WARNER, G.H. ,<u>78</u>, 6070-6071, (1956)].
La préparation des amines propargyliques de formule (**III**) peut être réalisée selon la méthode de HENNION, G.F., BOISELLE, A.P.,J. Am. Chem. Soc., <u>26</u>, 725-727, (1961). Comme cela est présenté dans la figure 4, cette méthode consiste en une chloration de l'alcool tertiaire de formule (**V**) en milieu acide chlorhydrique en présence de cuivre et chlorure cuivreux avec un rendement voisin de 70%.
L'alcool peut être préparé par exemple à partir de la cétone de formule $R_2COR_3$ et d'un acétylure.
Le chlorure propargylique de formule (**VI**) est ensuite soumis à l'action de l'amine de formule $R_5NHR_6$ selon [HENNION, G.F., NELSON, K.W., J. Am. Chem. Soc., <u>79</u>, 2142-2144, (1957) et HENNION, G.F., HANZEL, R.F.,J. Am. Chem. Soc., <u>82</u>, 4908-4912, (1960)].
Les rendements généralement obtenus se situent entre 20% et 60%.

Les composés de formule (**I**) ($R_1 = N^+(R_5R_6R_7)$, $X^-$) selon l'invention peuvent être synthétisés selon le schéma réactionnel de la figure-5. Le procédé de synthèse consiste à faire réagir l'halogénure $R_7X$ (de préférence l'iodure) ou bien l'acide minéral ou organique ($R_7$ = H) sur le composé de formule (**I**) ($R_1 = N(R_5R_6)$. Les rendements sont voisins de 70%.

Ainsi, alors que la synthèse des composés de la demande de brevet WO-98/44919 nécessitait une étape délicate de déprotection de l'amine propargylique qui empêchait une préparation à grande échelle de ces composés, la synthèse des composés de la présente invention présente l'avantage d'être plus simple et de pouvoir être développée à grande échelle, avec de meilleurs rendements.

Ces nouveaux composés présentent en outre l'avantage d'être plus stables et surtout beaucoup plus actifs dans l'inhibition de la croissance des cellules transformées résistantes à l'apoptose du fait de la surexpressibn du gène $bcl_2$ que ceux décrits dans la demande de brevet WO-98/44919, tels que le thioampal.

Les composés selon l'invention ont également l'avantage d'être actifs dans l'inhibition de la croissance des cellules transformées résistantes à l'apoptose non seulement du fait de la surexpression du gène $bcl_2$ mais également du fait de l'expression des enzymes ALDH (ALDH1, ALDH2, et/ou ALDH3), la surexpression du gène MDR « Multi Drug Resistant » ou du gène $BCL\text{-}X_L$, ce qui élargit leur champ d'utilisation.

Ainsi, la présente invention concerne également l'utilisation d'au moins un dérivé aminothiolester de formule (I) pour la préparation d'une composition pharmaceutique destinée à lever l'inhibition d'un caractère résistant à l'induction d'apoptose de cellules transformées, ce caractère étant dû au gène $bcl_2$, au gène MDR ou au gène $BCL\text{-}X_L$ présent dans ces cellules, ou à l'activité de ALDH.

La présente invention concerne également l'utilisation d'au moins un dérivé aminothiolester de formule (**I**) pour la préparation d'une composition pharmaceutique destinée à lever l'inhibition du caractère résistant à la chimiothérapie, ou à la radiothérapie ou aux antiandrogènes de cellules transformées.

Plus particulièrement, le caractère résistant à la chimiothérapie ou aux antiandrogènes de cellules transformées est dû au gène $bcl_2$, au gène $BCL\text{-}X_L$ ou au gène MDR présent dans ces cellules ou à l'activité de l'enzyme ALDH. Plus particulièrement, le caractère résistant à la radiothérapie de cellules transformées est dû à la présence du gène $bcl_2$ ou au taux élevé de glutathion dans ces cellules.

En effet, les enzymes aldéhyde déshydrogénases (ALDH) catalyse l'oxydation de différents aldéhydes aliphatiques et aromatiques en leur acide carboxylique correspondant en présence du cofacteur NAD ou NADP. Ces enzymes de différentes sous-familles et notamment ALDH1, ALDH2 ou ALDH3, sont présentent dans différents organes et jouent un rôle dans la détoxification des xenobiotiques. L'augmentation de l'activité de ces enzymes provoque une résistance aux agents anticancéreux tel que le cyclophosphamide [MAGNI et al., Blood, 87, 1097-1103, (1996)]. Comme indiqué dans la demande de brevet WO98/44919, l'utilisation d'un inhibiteur de l'activité de l'enzyme ALDH1 permet de lever l'inhibition du caractère résistant à la chimiothérapie ou aux antiandrogènes induit par ALDH1.

Il existe, au sein d'une tumeur cancéreuse, certaines cellules cancéreuses dites Multi Drug Resistant (MDR) qui ont un caractère résistant aux agents chimiothérapeutiques. Parmi celles-ci, les cellules R7 surexprimant le gène MDR ont été décrites comme étant résistantes à la daunorubicine [JEANNESSON, P. et al., Cancer Res., 50, 1231-1236, (1990)].

La lignée cellulaire LY-ar, cellules de lymphomes de souris, a été décrite comme étant résistante aux radiations, contrairement aux cellules LY-as qui elles sont sensibles à la radiothérapie [MIRKOVIC, N. et al., Oncogene, 15, 1461-1470, (1997)]. Cette résistance est due à la surexpression du gène $bcl_2$.

Ces composés présentent en outre l'avantage d'induire l'apoptose d'une grande variété de cellules transformées, ce qui permet de les utiliser dans la préparation de compositions pharmaceutiques destinées au traitement d'un grand nombre de pathologies cancéreuses mais également d'autres maladies, plus particulièrement les maladies liées à une hyperprolifération cellulaire, telles que les maladies auto-immunes ou les allergies.

Bien qu'induisant sélectivement l'apoptose des cellules transformées, ces composés présentent en outre, dans certaines gammes de concentrations plus élevées, des propriétés d'induction d'apoptose dans des cellules normales, telles que les cellules MRC5. Ceci permet de les utiliser dans la préparation de compositions cosmétiques destinées notamment à la prévention ou au traitement du vieillissement chronologique ou photo-induit.

L'objet de la présente invention concerne également les composés de formule (I) ci-dessus à titre de médicament. Les composés selon l'invention conviennent particulièrement bien dans les domaines de traitement suivants :

1) dans le traitement ou la prévention des états cancéreux ou précancéreux,

2) pour traiter les affections dermatologiques liées à un désordre de la kératinisation portant sur la différenciation, et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,

3) pour traiter les ichtyoses, les états ichtyosiformes, les kératodermies palnioplantaires, les leucoplasies et les états leucoplasiformes.

4) pour traiter des affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale,

5) pour traiter les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que les lésions précancéreuses cutanées telles que les kératoacanthomes,

6) pour traiter les dermatoses immunes telles le lupus érythémateux, les maladies immunes bulleuses et les maladies du collagène, telle la sclérodermie,

7) dans le traitement d'affections dermatologiques ou générales à composante immunologique,

8) pour lutter contre les troubles de la fonction sébacée tels que l'hyperséborrhée de l'acné ou la séborrhée simple,

9) dans le traitement de désordres cutanés dus à une exposition aux rayonnements U.V. ainsi que pour réparer

ou lutter contre le vieillissement de la peau, qu'il soit photo-induit ou chronologique ou pour réduire les pigmentations et les kératoses actiniques, ou toutes pathologies associées au vieillissement chronologique ou actinique,

10) pour prévenir ou traiter les troubles de la cicatrisation ou pour prévenir ou pour réparer les vergetures,

11) dans le traitement d'affections inflammatoires telles que l'arthrite,

12) dans le traitement de toute affection d'origine virale au niveau cutané ou général, telle que le syndrome de Kaposis ou les hépatites, et

13) pour traiter certains troubles ophtalmologiques, notamment les coméopathies.

Dans les domaines thérapeutiques mentionnés ci-dessus, les composés selon l'invention peuvent être avantageusement employés en combinaison avec des composés 3-méthylthiopropanoylthio-esters de l'acide thioctique, le méthional, de nombreux agents antinéoplasiques, des rétinoides, avec des corticostéroïdes ou des oestrogènes, en association avec des anti-oxydants, avec des α-hydroxy ou α-céto acides ou leurs dérivés, avec des bloqueurs de canaux potassiques, en association avec d'autres médicaments connus pour interférer avec le système immunitaire (par exemple la cyclosporine, le FK 506, les glucocorticoïdes, les anticorps monoclonaux, les cytokines ou les facteurs de croissance), en association avec des dérivés de la vitamine D ou avec des composés analogues de la vitamine D.
Parmi les agents antinéoplasiques, on peut notamment citer la dexamethasone, la cyclophosphamide, le cisplatin, l'étoposide et le BCNU (N,N-Bis(2-chloroéthyl)-N-nitrosourea), qui sont également capables d'induire l'apoptose.
Parmi les 3-méthylthiopropanoylthio-esters de l'acide thioctique on peut notamment citer les composés décrits dans la demande de brevet EP 947503 et plus particulièrement le composé 21, c'est à dire l'iodure de 6-S, 8-S bis (3-méthylthiopropanoyl) octanoate de (2'-triméthylammonium éthyle) désigné ci-après par Metmetlico.
Il a en effet été mis en évidence un effet de synergie des composés de l'invention dans leur activité anti-tumorale lorsqu'ils sont utilisés en association avec un agent thérapeutique antitumoral tel que décrit dans la demande de brevet EP 947503.
Par rétinoides, on entend des ligands des récepteurs RAR ou RXR, de type agonistes ou antagonistes, naturels ou synthétiques.
Parmi les vitamines D ou leurs dérivés, on peut notamment citer les dérivés de la vitamine $D_2$ ou $D_3$ et en particulier la 1,25-dihydroxyvitamine D3, les composés analogues de la vitamine D choisi parmi les composés décrits dans les demandes de brevet FR 98/13747, FR 99/14783 ou FR 99/14781.
Parmi les antioxydants, on peut notamment citer l'α-tocophérol, la Super Oxyde Dismutase, l'Ubiquinol ou certains chélatants de métaux.
Parmi les α-hydroxy ou α-céto acides ou leurs dérivés, on peut notamment citer la cétoleucine, la cétoisoleucine, la cétovaline, le 2-oxobutyrate, l'acide 4-méthylthio-2-oxobutanoïque, les acides lactique, malique, citrique, glycolique, mandélique, tartrique, glycérique, ou leurs sels, amides ou esters.
Parmi les bloqueurs de canaux potassiques, on peut notamment citer le Minoxidil (2,4-diamino-6-pipéridino-pyrimidine-3-oxyde) et ses dérivés.
La demanderesse a également constaté de façon inattendue et surprenante une activité synergique due à l'association de deux composés selon l'invention.
La présente invention a également pour objet de nouvelles compositions pharmaceutiques ou cosmétiques contenant, en tant qu'agent actif, une quantité efficace d'au moins un nouveau composé objet de l'invention dans un excipient physiologiquement acceptable.
La présente invention a donc aussi pour objet une telle composition pharmaceutique destinée notamment au traitement des affections susmentionnées.
La présente invention concerne également de nouvelles compositions pharmaceutiques pour le traitement, la régression et la prévention du cancer contenant, en tant qu'agent thérapeutique antitumoral, une quantité efficace d'au moins un nouveau composé objet de la présente invention seul ou en association avec un agent thérapeutique antitumoral choisi parmi ceux décrits dans la demande de brevet EP 947503.
L'administration des composés selon l'invention peut être effectuée par voie entérale, parentérale, topique ou oculaire.
Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.
Par voie parentérale, la composition peut se présenter sous forme de solutions ou suspensions pour perfusion ou pour injection.

Les composés de formule (I) selon l'invention sont généralement administrés à une dose journalière d'environ 0,001 mg/kg à 100 mg/kg en poids corporel en 1 à 3 prises.

Par voie topique, la composition cosmétique ou pharmaceutique à base de composés selon l'invention est plus particulièrement destinée au traitement de la peau du cuir chevelu et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques ou d'hydrogels permettant une libération contrôlée. Elle peut en variante se présenter sous la forme de shampoing ou d'après-shampoing ou de savon.

Ces compositions par voie topique peuvent se présenter sous forme anhydre, sous forme aqueuse ou sous forme d'émulsion selon l'indication thérapeutique.

Par voie oculaire, ce sont principalement des collyres.

Pour une application pharmaceutique, les composés de formule (I) sont utilisés par voie topique ou oculaire à une concentration généralement comprise entre 0,0001 % et 10 % en poids, de préférence entre 0,001 et 1 % en poids, par rapport au poids total de la composition.

Les composés de formule (**I**) selon l'invention trouvent également une application dans le domaine cosmétique, en particulier dans l'hygiène corporelle et capillaire et notamment pour le traitement des peaux à tendance acnéique, pour le traitement ou la prévention des vergetures, dans la protection contre les effets néfastes du soleil, pour prévenir et/ou lutter contre le vieillissement photo-induit ou chronologique, ou pour lutter contre l'aspect gras de la peau ou des cheveux.

La concentration en composé de formule (I) dans les compositions cosmétiques peut être comprise entre 0,001 et 3 % en poids par rapport au poids total de la composition.

Les compositions pharmaceutiques ou cosmétiques selon l'invention peuvent, en outre, contenir des additifs inertes ou même pharmacodynamiquement ou cosmétiquement actifs ou des combinaisons de ces additifs et notamment: des agents mouillants; des agents dépigmentants tels que l'hydroquinone, l'acide azelaïque, l'acide caféique ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzylcystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione); des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, ou des filtres UV-A et UV-B.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples d'obtention de composés actifs de formule (I) selon l'invention ainsi que des exemples de tests d'évaluation de l'activité biologique des composés de formule (I) selon l'invention.

## A. EXEMPLES DE COMPOSES

### EXEMPLE 1

Procédé de préparation du 4-diméthylamino-4-méthyle-pent-2-ynethioate de S-méthyle

A une solution de 0.855g de 3-diméthylamino-3-méthyl-but-1-yne [HENNION, G.F., NELSON, K.W. (1957) J. Am. Chem. Soc., 79, 2142-2144 ] (2.80 mmol) dans 14 ml de tétrahydrofuranne, on ajoute en 5 minutes à -70°C 4.07 ml (9.24 mmol) d'une solution 2.27 M de *n*butyllithium dans l'hexane. Après 5 minutes le milieu réactionnel est ramené à 0°C et agité encore 30 minutes à cette température. Après retour à -70°C, on cannule 2 ml d'oxysulfure de carbone préalablement condensé et on agite 30 minutes à -70°C. Le milieu réactionnel est ensuite porté à 0°C en 30 minutes puis on ajoute 0.575 ml (9.24 mmol) d'iodure de méthyle et on poursuit l'agitation pendant 2 heures à 0°C. On dilue dans 250 ml d'éther, on lave par une solution saturée de chlorure de sodium (3x30 ml), on sèche sur sulfate de sodium. Après évaporation sous vide et purification par chromatographie sur gel de silice (élution par un mélange éther de

pétrole / acétate d'éthyle = 70 / 30) on isole 1.16g du composé exemple 1 sous forme d'huile incolore (rendement : 81%)
$^1$H RMN (300 MHz, $CDCl_3$) : ν= 1.42 (s, 6H, $(CH_3)_2C$), 2.31 (s, 6H, $(CH_3)_2N$), 2.39 (s, 3H, $CH_3S$).

**EXEMPLE 2**

Procédé de préparation de l'iodure de 4-méthyle-4-triméthylammonium-pent-2-ynethioate de S-méthyle

A 0.184 g (0.99 mmol) du composé obtenu à l'exemple 1 dans 10ml d'acétate d'éthyle à température ambiante est ajouté 0.25 ml (4.02 mmol) d'iodure de méthyle. Le mélange est laissé pendant 4 jours à l'obscurité. On évapore sous vide et on purifie par chromatographie sur gel de silice (élution par un mélange dichlorométhane / méthanol = 90 /10). On isole ainsi 0.229 g du composé exemple 2 (rendement 70%)sous forme de solide.
$^1$H RMN (300 MHz, $CDCl_3$):ν = 1.96 (s, 6H, $(CH_3)_2C$), 2.46 (s, 3H, $CH_3S$), 3.62 (s, 6H, $(CH_3)_3N$).

**EXEMPLE 3**

Procédé de préparation du 4-di-*n*-propylamino-4-méthyle-pent-2-ynethioate de S-méthyle

Préparation identique à celle décrite à l'exemple 1 en utilisant la 3-di-*n*-propylamino-3-méthyl-but-1-yne [HENNION, G.F., HANZEL, R.F., J. Am. Chem. Soc.,82, 4908-4912, (1960)] à la place de la 3-diméthylamino-3-méthyl-but-1-yne. Echelle : 2.8 mmol, purification par chromatographie sur gel de silice (éluant éther de pétrole / acétate d'éthyle = 95 / 5), rendement : 75%. Huile incolore.
$^1$H RMN (300 MHz, $CDCl_3$):ν = 0.86 (t, J = 7.36, 6H, $CH_3$-$CH_2$) 1.46 (s, 6H, $(CH_3)_2C$), 1.47 (m, 4H, $CH_3$-$CH_2$) 2.38 (s, 3H, $CH_3S$), 2.52 (m, 4H, $CH_2N$).

**EXEMPLE 4**

Procédé de préparation du 4-méthyl-4-pyrrodin-1-yl-pent-2-ynethioate de S-méthyle

Préparation identique à celle décrite à l'exemple 1 en utilisant la 3-méthyl-3-pyrrodin-1-yl-but-1-yne [HENNION, G.F., NELSON, K.W., J. Am. Chem. Soc., 79, 2142-2144, (1957)] à la place de la 3-diméthylamino-3-méthyl-but-1-yne. Echelle : 2.8 mmol, purification par chromatographie sur gel de silice (éluant éther de pétrole / acétate d'éthyle = 70 / 30), rendement : 85%. Huile incolore.
$^1$H RMN (300 MHz, $CDCl_3$) :ν = 1.45 (s, 6H, $(CH_3)_2C$), 1.81 (m, 4H, $NCH_2$-$CH_2$) 2.39 (s, 3H, $CH_3S$), 2.72 (m, 4H, $CH_2N$).

**EXEMPLE 5**

Procédé de préparation du 4-méthyl-4-morpholin-4-yl-pent-2-ynethioate de S-méthyle

Préparation identique à celle décrite à l'exemple 1 en utilisant la 3-di-*n*-propylamino-3-méthyl-but-1-yne [HENNION, G.F., HANZEL, R.F., J. Am. Chem. Soc.,82, 4908-4912, (1960)] à la place de la 3-méthyl-3-morpholin-4-yl-but-1-yne. Echelle : 1.5 mmol, purification par chromatographie sur gel de silice (éluant éther de pétrole / acétate d'éthyle = 60 / 40), rendement : 77%. Solide blanc.
$^1$H RMN (300 MHz, $CDCl_3$):ν = 1.43 (s, 6H, $(CH_3)_2C$), 2.40 (s, 3H, $CH_3S$), 2.65 (m, 4H, $CH_2O$), 3.97 (m, 4H, $CH_2N$).

**B. EXEMPLES DE TESTS D'EVALUATION DE L'ACTIVITE BIOLOGIQUE DES COMPOSES DE L'INVENTION**

**Exemple 1- Effet des composés sur la croissance des cellules transformées ou non transformées**

**a) Effet des composés sur la croissance des cellules DU145 et BAF3 $bcl_2$**

Les cellules utilisées correspondent à deux lignées cellulaires : des cellules métastatiques de cancer de la prostate humaine (DU145), et des cellules lymphoïdes murines transfectées par le gène $bcl_2$ humain (BAF3 $bcl_2$).
Le mode opératoire utilisé pour les tests sur le DU145 est le suivant : $10^5$ cellules sont incubées dans les différents puits d'une microplaque dans 1ml de milieu de culture.
Les composés sont ajoutés 4 heures après dépôt des cellules dans les puits.
Après 72 heures, les cellules sont prélevées à différents temps. Les cellules DU145 sont lavées deux fois au PBS et récupérées directement avec du chlorure de sodium à 0.1M.
L'effet sur la croissance des cellules DU145 est mesurée par le dosage des protéines selon la méthode de Lowry

(LOWRY, O. H., ROSENBROUGH, N.J., FARR, A.L. and RANDALL, J. Biol. Chem. 193, 265-275, (1951)) et de l'ADN par la méthode de Hoechst (WEST, D.C., SATTAR, A. and KUMAR, S., Anal. Biochem. 147, 289-295, (1985)).

Le mode opératoire utilisé pour les tests sur les cellules BAF3 $bcl_2$ est le suivant :

$10^5$ cellules sont incubées dans les différents puits d'une microplaque dans 1ml de milieu de culture. Après 4 heures d'incubation, les composés à tester sont ajoutés dans les puits contenant les cellules BAF3 $bcl_2$.

Pour les cellules BAF3 $bcl_2$, la croissance est mesurée par le dénombrement des cellules viables en présence de Bleu Trypan à 0.1 %. Le témoin est constitué par le thioampal, décrit dans la demande de brevet WO-98/44919.

Les résultats sont rassemblés dans le tableau I ci-dessous :

**Tableau I**

| | | IC50 (µM) | |
|---|---|---|---|
| | | DU145 | BAF3 $bcl_2$ |
| **Exemple comparatif** | Thioampal | 650 | 100 |
| **Composés de l'invention** | Exemple 1 | 5.9 | 0.25 |
| | Exemple 3 | 8 | 2.7 |
| | Exemple 4 | 7.2 | 2.7 |
| | Exemple 5 | 8.8 | 12.0 |

La concentration $IC_{50}$ est la concentration correspondant à une inhibition de 50% de la croissance des cellules.

Ces résultats montrent que les valeurs de $IC_{50}$ des composés de l'invention sont très inférieures à celle obtenue pour le thioampal. Ainsi, l'inhibition de la croissance des cellules DU145 et des cellules BAF3 $bcl_2$ obtenue avec les composés selon l'invention est plus forte que l'inhibition obtenue avec le thioampal.

Cette inhibition de la croissance des cellules DU145 et des cellules BAF3 $bcl_2$ est due au moins en partie à une augmentation des phénomènes d'apoptose de ces cellules.

Ainsi, ces résultats suggèrent que les composés de l'invention induisent davantage l'apoptose des cellules DU145 et des cellules BAF3 $bcl_2$ que le thioampal.

**b) Effet des composés sur la croissance des cellules L1210, L1210T, B16 et MRC5**

Les cellules utilisées correspondent à différentes lignées cellulaires : des fibroblastes embryonnaires normaux de poumon humain (MRC5), des cellules de mélanome de souris (B16), des cellules de leucémie lymphocytaire de souris (L1210) et des cellules L1210T obtenues par infection des cellules L1210 par un vecteur retroviral portant le ADNc humain ALDH1.

Le mode opératoire utilisé pour les tests sur les cellules L1210 est le suivant : $10^5$ cellules sont incubées dans les différents puits d'une microplaque dans 1ml de milieu de culture. Après 4 heures d'incubation, les composés à tester sont ajoutés dans les puits contenant les cellules L1210.

Pour les cellules L1210, la croissance est mesurée par le dénombrement des cellules viables en présence de Bleu Trypan à 0.1%.

Le mode opératoire utilisé pour les tests sur les cellules B16 et MRC5 est le suivant :

Les composés à tester sont ajoutés 4 heures après dépôt des cellules dans les puits. Après 72 heures, les cellules sont prélevées à différents temps. Les cellules B16 et MRC5 sont lavées deux fois au PBS et récupérées directement avec du chlorure de sodium à 0.1M.

L'effet sur la croissance des cellules B16 et MRC5 est mesurée par le dosage des protéines selon la méthode de Lowry (LOWRY, O. H., ROSENBROUGH, N.J., FARR, A.L. and RANDALL,J. Biol. Chem. 193, 265-275, (1951)) et de l'ADN par la méthode de Hoechst (WEST, D.C., SATTAR, A. and KUMAR, S., Anal. Biochem. 147, 289-295, (1985)).

Les résultats sont rassemblés dans le tableau II ci-dessous :

**Tableau II**

| | | IC50 (µM) | | | |
|---|---|---|---|---|---|
| | | L1210 | L1210T | B16 | MRC5 |
| Exemple comparatif | Thioampal | 90 | 140 | NT | >600 |
| Composé de l'invention | Exemple 1 | 1.2 | 1.6 | 0.8 | 8.4 |

NT signifie non testé

La concentration $IC_{50}$ est la concentration correspondant à une inhibition de 50% de la croissance des cellules.

Ces résultats montrent que les valeurs de $IC_{50}$ des composés de l'invention sont très faibles et très inférieures à celles obtenues pour le thioampal. Ainsi, l'inhibition de la croissance des cellules L1210, L1210T, B16 et MRC5 obtenue avec les composés selon l'invention est importante et beaucoup plus forte que l'inhibition obtenue avec le thioampal.

Cette inhibition de la croissance des cellules L1210, L1210T, B16 et MRC5 est due au moins en partie à une augmentation des phénomènes d'apoptose de ces cellules. Ainsi, ces résultats suggèrent que les composés de l'invention induisent davantage l'apoptose des cellules L1210, L1210T, B16 et MRC5 que le thioampal.

**2- Mesure d'induction d'apoptose dans les cellules BAF3-b0 et BAF3-$bcl_2$ par les composés de l'invention**

Les cellules BAF3-$bcl_2$ correspondent à des cellules BAF3 (cellules lymphocytaires de souris) transfectées par le gène $bcl_2$. Parmi celles-ci, quatre lignées appelées H16, G18, B14 et G21 surexpriment le gène $bcl_2$.

Dans la suite, les cellules dénommées BAF3-b0 correspondent à des cellules BAF3 non transfectées par le gène $bcl_2$.

Alors que les cellules BAF3-b0 subissent l'apoptose (plus de 80% des cellules) en l'absence l'interleukine 3 (IL3) en 16 heures [d'après COLLINS, M.K.L., MARVEL, J., MALDE, P. & LOPEZ-RIVAS, A., J. Exp. Med. 176, 1043-1051, (1992)], les cellules BAF3-$bcl_2$ ne présentent aucun signe d'apoptose en l'absence d'IL3. Elles sont donc résistantes à l'apoptose.

Le mode opératoire qui a été utilisé est le suivant :

Les cellules BAF3-b0 ou BAF3-$bcl_2$, mises en culture en présence d'IL3, ont été marquées par une méthode adaptée de celle décrite dans WRIGHT, S. et al., J. of Cell. Biochem. 48, 344-355, (1992).

$10^5$ cellules /ml ont été incubées avec 4.62 $KBq.ml^{-1}$ [$^3$H]-thymidine pendant 40 heures à 37°C. Après deux lavages avec un milieu de culture, 2,5. $10^6$ cellules ont été mises en culture en présence du composé à tester.

Après incubation pendant 24 heures, ces cellules ont été récupérées par centrifugation à 400g pendant 5 minutes et lavées 3 fois dans un tampon PBS. Les cellules récupérées dans le culot ont été lysées dans 2ml de 0,1% de Triton X-100, 20mM EDTA, 5mM Tris pH8 et centrifugées à 30000g à 4°C pendant 30 minutes.

Les surnageants ont été récupérés et les culots dissous dans 0,3 ml de 0,5 N NaOH.

Des aliquots du milieu de culture (1ml), du surnageant (0,3ml) et du culot solubilisé (0,1ml) ont été mis à doser dans un compteur à scintillation.

Le pourcentage de fragments d'ADN est calculé de la façon suivante :

$$\text{\% de fragments d'ADN} = \frac{\text{dpm du milieu de culture + dpm du surnageant}}{\text{dpm du milieu de culture + dpm du surnageant + dpm du culot solubilisé}}$$

***(dpm = désintégration par minute)***

Le pourcentage de fragmentation de l'ADN est une mesure directe de l'apoptose subie par les cellules.

Les résultats obtenus sont présentés dans le tableau III.

Tableau III

| | | | Clones $bcl_2$ | | | |
|---|---|---|---|---|---|---|
| | Exemple 1 (μM) | b0 | G21 | G18 | B14 | H16 |
| % expression de $bcl_2$ dans cellules BAF3 | 0 | 0 | 6.4 | 22.1 | 50.8 | 72 |
| Fragmentation de l'ADN (%) | 1 | 5.0 | 0 | 0.6 | 0 | 2.9 |
| | 2 | 10.6 | 5.8 | 7.3 | 2.0 | 6.3 |
| | 4 | 46.4 | 29.3 | 52.7 | 44.1 | 65.3 |
| | 8 | 71.1 | 71.8 | 84.3 | 90.0 | 94.2 |

Les cellules BAF3-b0 servent de témoins. L'apoptose de ces cellules est induite par ajout du composé exemple 1 et ce phénomène augmente de façon dose-dépendante.
Ces résultats montrent que la présence d'un composé selon l'invention permet de lever l'inhibition d'apoptose due au gène $bcl_2$ dans les lignées cellulaires H16, G18, B14 et G21 qui surexpriment le gène $bcl_2$.
Le mode opératoire de l'essai présenté dans le tableau IV ci-dessous est identique au précédent à la seule différence que le temps d'incubation est de 6 heures au lieu de 24 heures :

Tableau IV

| | % fragmentation de l'ADN des cellules $bcl_2$ H16 | |
|---|---|---|
| Conc (μM) | Thioampal | Exemple 1 |
| 50 | 0 | 2,15 |
| 100 | 1,6 | 0 |
| 200 | 7,6 | 30,4 |
| 400 | 16,4 | 83,8 |
| 600 | 26,5 | 81,5 |

L'apoptose de ces cellules est induite de manière beaucoup plus importante avec le composé exemple 1 qu'avec le thioampal, et ceci de façon dose-dépendante.
Ces résultats montrent que la présence du composé selon l'invention permet de lever l'inhibition d'apoptose due au gène $bcl_2$ et ceci de manière beaucoup plus forte que les composés de l'art antérieur.

**Exemple 2 : Effet des composés sur l'activité de ALDH1**

Le mode opératoire utilisé est indiqué ci-dessous :
260 mU de ALDH de levures de boulanger sont préincubées à 37 ou 0°C dans un mélange de 1 mM EDTA, 100 mM KCl, dans 60 mM de tampon phosphate de sodium pH 6 dans un volume final de 200 μl.
Dans chaque tube contenant les 200μl de mélange, le composé à tester est ajouté à la concentration de 400 μM, à l'exception d'une série de contrôle qui ne contient pas de composé à tester.
L'incubation effectuée à 0°C ou à 37°C est stoppée par l'addition de 100 μg de BSA (Bovin serum albumine) et de l'acétone à -20°C à une concentration finale de 80% pour précipiter les protéines (ALDH et BSA). Les tubes sont laissés à -20°C pendant 1 heure, puis centrifugés à 10000 rpm pendant 10 minutes, lavés avec de l'acétone à 80%.
Les protéines précipitées sont dissoutes dans 358 μl d'eau et ajoutées immédiatement au complexe réactionnel constitué 1 mM EDTA, 100 mM KCl, 2.35 mM NAD dans 60 mM de tampon phosphate de sodium pH 8.5.
La réaction est démarrée par l'addition de 2 mM de propanal et la densité optique (OD) est mesurée à 340 nm à T=0 et à T= 5, 10, 20 et 3 0 minutes.
L'activité de l'enzyme est mesurée par la variation de densité optique Δ OD par minute (QUASH G. et al., *Enzymology and molecular biology of carbonyl metabolism*, Weiner et al. eds., Kluwer Academic/Plenum Publishers, New-York, 97-106).
Les résultats obtenus sont présentés sur la figure 6.
Le pourcentage d'activité (A%) de l'enzyme ALDH1 obtenu à différents temps de préincubation au composé exemple 1 à 0°C (représenté par ■) ou 37°C (représenté par ▲) ou bien sans préincubation au composé exemple 1 à 0°C

(représenté par ❑) ou à 37°C (représenté par Δ) est mesuré en fonction du temps t (exprimé en minutes).

Lorsque la préincubation par le composé exemple 1 est effectuée à la température de 0°C, il n'y a pas d'effet d'inhibition du composé exemple 1 sur l'activité de l'enzyme. En effet les deux courbes obtenues avec ou sans préincubation au composé exemple 1 sont superposables. Ceci s'explique par le fait qu'à cette température, le composé exemple 1 n'a pas pu être métabolisé par l'ALDH1. De ce fait, la formation de liaisons covalentes entre le principe actif formé après clivage et l'enzyme n'a pas pu s'établir.

En revanche, lorsque la préincubation par le composé exemple 1 est effectuée à la température de 37°C, l'enzyme ALDH1 métabolise le composé exemple 1 et se lie à lui par des liaisons covalentes, et on observe un effet d'inhibition de l'activité de l'enzyme. Cet effet augmente en fonction du temps de préincubation par le composé exemple 1.

Ces résultats montrent que les composés selon l'invention permettent de diminuer l'activité de ALDH, et présentent l'avantage d'être de type "suicide" (liaison covalente irréversible avec l'enzyme ALDH), permettant ainsi de lever la résistance aux agents anticancéreux.

**Exemple 3 : Effet des composés sur les cellules R7 surexprimant le gène MDR**

Les cellules K562 utilisées dans ce test ne surexpriment pas le gène MDR et servent de témoin, ce sont des cellules de leucémie myéloïde humaine. Contrairement aux cellules R7, cellules cancéreuses qui elles surexpriment le gène MDR.
L'objet du présent test est de mettre en évidence l'effet des composés de l'invention sur ces cellules R7.
Le mode opératoire utilisé est indiqué ci-dessous :
$10^5$ cellules ont été incubées dans les différents puits d'une microplaque dans 1ml de milieu de culture. Il s'agit d'une part de cellules R7 et d'autre part de cellules K562.
Après 4 heures d'incubation, le composé à tester (l'exemple 1 selon l'invention d'une part et la Daunorubicine d'autre part) a été ajouté dans les puits contenant les cellules.
Après 72 heures, les cellules ont été prélevées à différents temps.
La croissance des cellules a été mesurée par le dénombrement des cellules viables en présence de Bleu Trypan à 0.1%.
Les résultats sont présentés sur les figures 7 et 8.

Les figures 7 et 8 représentent respectivement le pourcentage d'inhibition (I%) par la daunorubicine et le composé exemple 1 de la croissance des cellules R7 et K562, obtenu à concentrations C croissantes. Les résultats obtenus avec les cellules K562 sont symbolisés par ♦ et ceux obtenus avec les cellules R7 par ▲.

Ces résultats montrent que le daunorubicine n'a aucun effet d'inhibition sur la croissance des cellules R7 surexprimant le gène MDR à des concentrations auxquelles elle inhibe la croissance des cellules K562.
En revanche, le composé exemple 1 inhibe de façon très importante la croissance des cellules R7. L'effet d'inhibition sur la croissance des cellules R7 est même supérieur à l'effet d'inhibition obtenu aux mêmes concentrations sur la croissance des cellules K562.
Ces résultats montrent que les composés selon l'invention permettent de lever la résistance à la chimiothérapie due au gène MDR.

**Exemple 4 : Effet de l'association d'un composé selon l'invention et d'un composé choisi parmi ceux décrits dans la demande de brevet EP 947503 sur la croissance des cellules DU145**

Dans la demande de brevet EP 947503, il a été décrit que des dérivés du méthional comprenant le méthional couplé par liaison thio-ester à l'acide thioctique présentaient une forte activité apoptotique sélective pour les cellules transformées et tumorales. Parmi ces composés, on peut citer l'iodure de 6-S, 8-S-bis (3-methylthiopropanoyl)octanoate de (2'-trimethylammonium éthyle) (composé 21 de ce document) désigné par metmetlico.

Le mode opératoire utilisé est indiqué ci-dessous :

Les cellules utilisées correspondent à des cellules métastatiques de cancer de la prostate humaine DU145.
$10^5$ cellules DU145 sont incubées dans les différents puits d'une microplaque dans 1ml de milieu de culture.
Les composés à tester sont ajoutés 4 heures après dépôt des cellules dans les puits. Après 72 heures, les cellules sont prélevées à différents temps. Les cellules sont lavées deux fois au PBS et récupérées directement avec du chlorure de sodium à 0.1M.
L'effet sur la croissance des cellules DU145 est mesurée par le dosage des protéines selon la méthode de Lowry (LOWRY, O. H., ROSENBROUGH, N.J., FARR, A.L. and RANDALL, J. Biol. Chem. 193, 265-275, (1951)) et de l'ADN par la méthode de Hoechst (WEST, D.C., SATTAR, A. and KUMAR, S., Anal. Biochem. 147, 289-295, (1985)).
Les produits testés sont le composé exemple 1, le metmetlico et leur mélange.
Les résultats sont rassemblés dans le tableau V.

Tableau V

| Composé (concentration) | % inhibition de la croissance des cellules DU145 |
|---|---|
| Exemple 1 (2µM) | 5.8 |
| Exemple 1 (3µM) | 16.1 |
| Exemple 1 (4µM) | 22.4 |
| Metmetlico (25µM) | 0 |
| Metmetlico (50µM) | 0 |
| Exemple 1 (2µM)+ Metmetlico (25µM) | 14.7 |
| Exemple 1 (2µM)+ Metmetlico (50µM) | 20.7 |
| Exemple 1 (3 µM)+ Metmetlico (25µM) | 26.9 |
| Exemple 1 (3 µM)+ Metmetlico (50µM) | 36.9 |
| Exemple 1 (4µM)+ Metmetlico (25µM) | 44.4 |
| Exemple 1 (4µM)+ Metmetlico (50µM) | 41.3 |

Les résultats montrent que le composé exemple 1 seul inhibe la croissance des cellules DU145. En revanche, le Metmetlico seul n'a pas d'effet inhibiteur sur la croissance de ces cellules DU145 aux concentrations utilisées dans cet exemple.

L'association d'un composé selon l'invention et d'un composé décrit dans la demande de brevet EP947503 inhibe de façon synergique la croissance des cellules DU145. Ces résultats démontrent la possibilité d'utiliser ces composés en association à des doses nettement inférieures de celles utilisables lorsqu'ils sont utilisés seuls.

**Exemple 5: Effet de l'association de deux composés selon l'invention sur la croissance des cellules DU145 ou des cellules normales**

$10^5$ cellules normales de prostate humaine ou $10^5$ cellules cancéreuses de prostate humaine (DU145) sont incubées dans les différents puits d'une microplaque dans 1ml de milieu de culture.
Les composés sont ajoutés 4 heures après dépôt des cellules dans les puits.
Après 72 heures, les cellules sont prélevées à différents temps. Les cellules DU145 ou les cellules normales sont lavées deux fois au PBS et récupérées directement avec du chlorure de sodium à 0.1M.
L'effet sur la croissance des cellules DU145 ou des cellules normales est mesurée par le dosage des protéines selon la méthode de Lowry (LOWRY, O. H., ROSENBROUGH, N.J., FARR, A.L. and RANDALL, J. Biol. Chem. 193, 265-275, (1951)) et de l'ADN par la méthode de Hoechst (WEST, D.C., SATTAR, A. and KUMAR, S., Anal. Biochem. 147, 289-295, (1985)).

Tableau VI

| Produit à tester (concentration) | % Inhibition des cellules normales de prostate humaine | % Inhibition des cellules DU145 |
|---|---|---|
| Exemple 1 (2µM) | 3.7 | 21.3 |
| Exemple 5 (2µM) | 1.3 | 25.2 |
| Exemple 5 (1µM) | 0 | 14.3 |
| Exemple 1 (2µM) + Exemple 5 (2µM) | 6.1 | 67.7 |
| Exemple 1 (2µM) + Exemple 5 (1µM) | 2.8 | 53.8 |

L'association de deux composés selon l'invention inhibe de façon synergique la croissance des cellules cancéreuses sans incidence notable sur la croissance des cellules normales, ce qui permet d'augmenter encore l'activité et la sélectivité des composés selon l'invention vis à vis des cellules transformées. Ces résultats démontrent la possibilité d'utiliser ces composés en association à des doses inférieures de celles utilisables lorsqu'ils sont utilisés seuls.

## C. EXEMPLES DE FORMULATION

| 1) | VOIE ORALE | |
|---|---|---|
| (a) | On prépare la composition suivante sous la forme d'un comprimé de 0,2 g | |
| | Composé de l'exemple 1<br>Amidon prégélatinisé<br>Cellulose microcristalline<br>Lactose<br>Stéarate de magnésium | 0,005 g<br>0,065 g<br>0,075 g<br>0,050 g<br>0,005 g |
| (b) | On prépare une suspension buvable, destinée à être conditionnée en ampoules de 5 ml | |
| | Composé de l'exemple 2<br>Glycérine<br>Sorbitol à 70 %<br>Saccharinate de sodium<br>Parahydroxybenzoate de méthyle<br>Arôme q.s.<br>Eau purifiée q.s.p. | 0,001 g<br>0,500 g<br>0,500 g<br>0,010 g<br>0,040 g<br><br>5 ml |
| (c) | On prépare la formulation suivante destinée à être conditionnée en gélules : | |
| | Composé de l'exemple 1<br>Composé de l'exemple 5<br>Cyclosporine<br>Amidon de maïs<br>Lactose q.s.p. | 0.01 mg<br>0,01 mg<br>0,050 g<br>0,060 g<br>0,300 g |

Les gélules utilisées sont constituées de gélatine, d'oxyde de titane et d'un conservateur.

| 2) | VOIE TOPIQUE | |
|---|---|---|
| (a) | On prépare la crème Eau-dans l'Huile non ionique suivante : | |
| | Composé de l'exemple 1<br>Mélange d'alcools de lanoline émulsifs, de cires et d'huiles raffinés, vendu par la Société BDF sous la dénomination "Eucérine anhydre"<br>Parahydroxybenzoate de méthyle<br>Parahydroxybenzoate de propyle<br>Eau déminéralisée stérile q.s.p. | 0,100 g<br>39,900 g<br><br>0,075 g<br>0,075 g<br>100,000 g |
| (b) | On prépare un gel en réalisant la formulation suivante : | |
| | Composé de l'exemple 1<br>Erythromycine base<br>Butylhydroxytoluène<br>Hydroxypropylcellulose vendue par la société Hercules sous le nom de "KLUCEL HF"<br>Ethanol (à 95°) q.s.p. | 0,001 g<br>4,000 g<br>0,050 g<br>2,000 g<br>100,000 g |
| (c) | On prépare une lotion en procédant au mélange des ingrédients suivants : | |
| | Composé de l'exemple 2<br>Propylène glycol<br>Butylhydxoxytoluène<br>Ethanol (à 95°) q.s.p. | 0,030 g<br>5,000 g<br>0,100 g<br>100,000 g |

(suite)

| | | |
|---|---|---|
| (d) | On prépare une composition cosmétique contre les effets néfastes du soleil en procédant au mélange des ingrédients suivants : | |
| | Composé de l'exemple 1 | 1,00 g |
| | Benzylidène camphre | 4,00 g |
| | Triglycérides d'acides gras | 31,00 g |
| | Monostéarate de glycérol | 6,00 g |
| | Acide stéarique | 2,00 g |
| | Alcool cétylique | 1,20 g |
| | Lanoline | 4,00 g |
| | Conservateurs | 0,30 g |
| | Propylène glycol | 2,00 g |
| | Triéthanolamine | 0,50 g |
| | Parfum | 0,40 g |
| | Eau déminéralisée q.s.p. | 100,00 g |
| (e) | On prépare la crème Huile dans l'Eau non ionique suivante : | |
| | Composé de l'exemple 3 | 0,500 g |
| | Acide rétinoique | 0,020 g |
| | Alcool cétylique | 4,000 g |
| | Monostéarate de glycérol | 2,500 g |
| | Stéarate de PEG 50 | 2,500 g |
| | Beurre de Karité | 9,200 g |
| | Propylène glycol | 2,000 g |
| | Parahydroxybenzoate de méthyle | 0,075 g |
| | Parahydroxybenzoate de propyle | 0,075 g |
| | Eau déminéralisée stérile q.s.p. | 100,000 g |
| (f) On prépare un gel topique en procédant au mélange des ingrédients suivants : | | |
| | Composé de l'exemple 4 | 0,050 g |
| | Ethanol | 43,000 g |
| | - tocophérol | 0,050 g |
| | Polymère carboxyvinylique vendu sous la dénomination "Carbopol 941 " par la société "Goodrich" | 0,500 g |
| | Triéthanolamine en solution aqueuse à 20 % en poids | 3,800 g |
| | Eau | 9,300 g |
| | Propylène glycol qsp | 100,000 g |
| (g) | On prépare une crème huile dans l'eau en réalisant la formulation suivante : | |
| | Composé de l'exemple 4 | 0,020 g |
| | 17-valérate de bétamethasone | 0,050 g |
| | S-carboxyméthyl cystéine | 3, 000 g |
| | Stéarate de polyoxyéthylène (40 moles d'oxyde d'éthylène) vendu sous le nom de "Myrj 52" par la société "ATLAS" | 4,000 g |
| | Monolaurate de sorbitan, polyoxyéthylène à 20 moles d'oxyded'éthylène vendu sous le nom de "Tween 20" par la société "ATLAS" | 1,800 g |
| | Mélange de mono et distéarate de glycérol vendu sous la dénomination de "Géléol" par la société "GATTEFOSSE" | 4,200 g |
| | Propylène glycol | 10,000 g |
| | Butylhydroxyanisole | 0,010 g |
| | Butylhydroxytoluène | 0,020 g |
| | Alcool cétostéarylique | 6,200 g |
| | Conservateurs | q.s. |

(suite)

| | | |
|---|---|---|
| (g) | On prépare une crème huile dans l'eau en réalisant la formulation suivante : | |
| | Perhydrosqualène | 18,000 g |
| | Mélange de triglycérides caprylique-caprique vendu sous la dénomination de "Miglyol 812" par la société "DYNAMIT NOBEL" | 4,000 g |
| | Triéthanolamine (99 % en poids) | 2,500 g |
| | Eau q.s.p. | 100,000 g |
| (h) On prépare la crème de type huile dans l'eau suivante : | | |
| | Acide lactique | 5,000 g |
| | Composé de l'exemple 2 | 0,020 g |
| | Stéarate de polyoxyéthylène (40 moles d'oxyde d'éthylène) vendu sous le nom de "Myrj 52" par la société "ATLAS" | 4,000 g |
| | Monolaurate de sorbitan, polyoxyéthylène à 20 moles d'oxyde d'éthylène vendu sous le nom de Tween 20" par la société "ATLAS" | 1,800 g |
| | Mélange de mono et distéarate de glycérol vendu sous la dénomination de "Geleol" par la société "GATTEFOSSE" | 4,200 g |
| | Propylène glycol | 10,000 g |
| | Butylhydroxyanisole | 0,010 g |
| | Butylhydroxytoluène | 0,020 g |
| | Alcool cétostéarylique | 6,200 g |
| | Conservateurs | q.s. |
| | Perhydrosqualène | 18,000 g |
| | Mélange de triglycérides caprylique-caprique vendu sous la dénomination de "Miglyol 812" par la société "DYNAMIT NOBEL" | 4,000 g |
| | Eau q.s.p. | 100,000 g |
| (i) On | prépare l'onguent anhydre suivant : | |
| | Composé de l'exemple 1 | 5,00 g |
| | Huile de vaseline | 50,00 g |
| | Butylhydrotoluène | 0,05 g |
| | vaseline blanche | qs 100 g |

| | | |
|---|---|---|
| 3) | VOIE INTRALESIONNELLE | |
| (a) | On prépare la composition suivante : | |
| | Composé de l'exemple 3 | 0,002 g |
| | Oléate d'éthyle qs | 10 g |
| (b) | On prépare la composition suivante : | |
| | Composé de l'exemple 1 | 0.05 % |
| | Polyethylene glycol | 20% |
| | Solution de NaCl à 0.9%qs | 100 |
| (c) | On prépare la composition suivante : | |
| | Composé de l'exemple 3 | 2.5 % |
| | Polyethylene glycol 400 | 20% |
| | Solution de NaCl à 0.9% | qs 100 |

| | | |
|---|---|---|
| 4) | VOIE INTRAVEINEUSE | |
| (a) | On prépare le composition injectable suivante: | |
| | Composé de l'exemple 1 | 0.001% |
| | Metmetlico | 0.01% |
| | Polyethylene glycol 400 | 20% |
| | Solution de NaCl à 0.9% | qs 100 |
| (b) | On prépare le composition injectable suivante: | |
| | Composé de l'exemple 2 | 0.01% |
| | Polyethylene glycol 400 | 20% |
| | Solution de NaCl à 0.9% | qs 100 |
| (c) | On prépare la composition de cyclodextrine suivante : | |
| | Composé de l'exemple 3 | 0,1 mg |
| | cyclodextrine | 0,10 g |
| | Eau pour injectable q.s.p. | 10,00 g |
| (d) | On prépare la composition de cyclodextrine suivante : | |
| | Composé de l'exemple 4 | 0,01 g |
| | 2-hydroxypropyl cyclodextrine | 0,10 g |
| | Eau pour injectable q.s.p. | 10,00 g |

**Revendications**

1. Composé, **caractérisé par le fait qu'**il correspond à la formule générale (**I**) suivante:

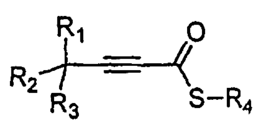

**(I)**

dans laquelle :

- $R_1$ représente:

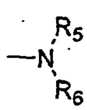 ou 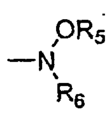 ou 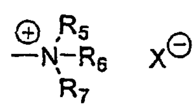 ou 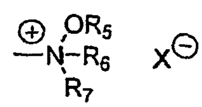

- $R_2$ et $R_3$, indépendamment, représentent un radical alkyle ayant de 1 à 7 atomes de carbone, saturé ou insaturé, cyclique, linéaire ou ramifié ou un radical aryle ayant de 1 à 7 atomes de carbone, ou un radical aralkyle ayant de 1 à 12 atomes de carbone ou pris ensemble forment un cycle alkyle saturé ayant de 3 à 7 atomes de carbone,
- $R_4$ représente un radical alkyle ayant de 1 à 7 atomes de carbone saturé ou insaturé, cyclique, linéaire ou ramifié ou un radical aryle ayant de 1 à 7 atomes de carbone ou un radical aralkyle ayant de 1 à 12 atomes de carbone,
- $R_5$ et $R_6$, indépendamment, représentent un radical alkyle ayant de 1 à 7 atomes de carbone saturé ou insaturé, cyclique, linéaire ou ramifié ou un radical aryle ayant de 1 à 7 atomes de carbone, ou un radical aralkyle ayant de 1 à 12 atomes de carbone ou pris ensemble forment avec l'atome d'azote un hétérocycle azoté saturé ou insaturé ayant de 2 à 6 atomes de carbone, éventuellement substitué par un oxygène, un soufre ou par un atome d'azote éventuellement substitué par un radical alkyle ayant de 1 à 7 atomes de carbone saturé ou

insaturé, cyclique linéaire ou ramifié ou un radical aryle ayant de 1 à 7 atomes de carbone ou un radical aralkyle ayant de 1 à 12 atomes de carbone,

- $R_7$ représente un atome d'hydrogène, un radical alkyle ayant de 1 à 7 atomes de carbone saturé ou insaturé cyclique, linéaire ou ramifié ou un radical aryle ayant de 1 à 7 atomes de carbone, ou un radical aralkyle ayant de 1 à 12 atomes de carbone et
- X représente un ion halogénure, ou un anion nitrate, sulfate, sulfonate, carboxylate, thiocyanate, ou phosphate.

**2.** Composé selon la revendication 1, **caractérisé en ce que** le radical alkyle cyclique ayant de 1 à 7 atomes de carbone est choisi parmi le radical cyclopropyle, cyclopentyle, cyclohexyle ou cycloheptyle.

**3.** Composé selon l'une quelconque des revendications précédentes, **caractérisé en ce que** le radical alkyle saturé linéaire ou ramifié est choisi parmi le radical méthyle, éthyle, *n*-propyle, *i*-propyle, *n*-butyle, 2-butyle, *t*-butyle, *n*-pentyle, 2-pentyle, *i*-pentyle, *n*-hexyle, 2-hexyle, 3-hexyle, *n*-heptyle, 2-heptyle, 3-heptyle ou *i*-heptyle.

**4.** Composé selon l'une quelconque des revendications précédentes, **caractérisé en ce que** le radical aryle est choisi parmi le radical phényle, tolyle, chlorophényle, nitrophényle, méthoxyphényle, thiophène ou furane.

**5.** Composé selon l'une quelconque des revendications précédentes, **caractérisé en ce que** le radical hétérocycle azoté est choisi parmi la pyrrolidine, la pipéridine, la morpholine, la thiamorpholine, la pipérazine, l'homopipérazine ou la N-méthyl pipérazine.

**6.** Composé selon l'une quelconque des revendications précédentes, **caractérisé en ce que** le radical alkyle insaturé ayant de 1 à 7 atomes de carbone est choisi parmi le radical allyle ou vinyle.

**7.** Composé selon l'une quelconque des revendications précédentes, **caractérisé en ce que** le radical aralkyle ayant de 1 à 12 atomes de carbone est choisi parmi le radical benzyle éventuellement substitué par un radical alkyle ayant de 1 à 5 atomes de carbone, les radicaux 1-phényl éthyle, 1-phényle propyle, 1-phényle butyle, 1-phényle pentyle.

**8.** Composé selon l'une quelconque des revendications précédentes, **caractérisé en ce que** l'ion halogénure est choisi parmi le fluorure, le chlorure le bromure ou l'iodure.

**9.** Composé selon la revendication 1, **caractérisé en ce qu'**il est choisi parmi :

- le 4-diméthylamino-4-méthyle-pent-2-ynethioate de S-méthyle ;
- l'iodure de 4-méthyle-4-triméthylammonium-pent-2-ynethioate de S-méthyle ;
- le 4-di-*n*-propylammino-4-méthyle-pent-2-ynethioate de S-méthyle ;
- le 4-méthyl-4-pyrrodin-1-yl-pent-2-ynethioate de S-méthyle ;
- et le 4-méthyl-4-morpholin-4-yl-pent-2-ynethioate de S-méthyle.

**10.** Composé selon la revendication 1, **caractérisé en ce qu'**il présente l'une au moins des caractéristiques suivantes:

- $R_2$ et $R_3$, représentent un radical méthyle ;
- $R_4$ représente un radical méthyle ;
- $R_5$ et $R_6$, indépendamment, représentent un radical alkyle ayant de un à trois atomes de carbone ou pris ensemble forment avec l'atome d'azote un hétérocycle azoté choisi parmi la pyrrolidine, la pipéridine ou là morpholine ;
- $R_7$ représente un radical méthyle ou un atome d'hydrogène ;
- et X désigne un ion iodure, un ion formiate ou un ion carboxylate.

**11.** Composé selon l'une quelconque des revendications 1 à 10 à titre de médicament.

**12.** Utilisation d'un composé selon l'une quelconque des revendications 1 à 10 pour la fabrication d'un médicament destiné au traitement :

1) des états cancéreux ou précancéreux,
2) des affections dermatologiques liées à un désordre de la kératinisation portant sur la différenciation, et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle, et/ou
3) des ichtyoses, des états ichtyosiformes, des kératodermies palmoplantaires, des leucoplasies et des états leucoplasiformes, et/ou
4) des affections dermatologiques avec une composante immuno-allergique inflammatoire, avec ou sans trouble de la prolifération cellulaire, et notamment le psoriasis, le rhumatisme psoriasique, l'eczéma, l'atopie respiratoire et l'hypertrophie gingivale, et/ou
5) des proliférations dermiques ou épidermiques bénignes ou malignes, d'origine virale ou non, telles que les verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que les lésions précancéreuses cutanées telles que les kératoacanthomes, et/ou
6) des dermatoses immunes telles le lupus érythémateux, des maladies immunes bulleuses et des maladies du collagène, telle que la sclérodermie, et/ou
7) d'affections dermatologiques ou générales à composante immunologique, et/ou
8) des troubles de la fonction sébacée tels que l'hyperséborrhée de l'acné ou la séborrhée simple, et/ou
9) des désordres cutanés dus à une exposition aux rayonnements U.V., du vieillissement photo-induit ou chronologique de la peau ou des pigmentations et des kératoses actiniques, ou des pathologies associées au vieillissement chronologique ou actinique, et/ou
10) des troubles de la cicatrisation ou des vergetures, et/ou
11) d'affections inflammatoires telles que l'arthrite, et/ou
12) d'affections d'origine virale au niveau cutané ou général, telles que le syndrome de Kaposis ou les hépatites, et/ou
13) des troubles ophtalmologiques, notamment les coméopathies.

**13.** Utilisation d'un composé selon l'une quelconque des revendications 1 à 10 pour la fabrication d'un médicament destiné au traitement des états cancéreux ou précancéreux.

**14.** Composition, **caractérisée en ce qu'**elle comprend, dans un support physiologiquement acceptable, au moins l'un des composés selon l'une quelconque des revendications 1 à 10.

**15.** Composition selon la revendication 14, **caractérisée par le fait qu'**elle contient le composé actif en une quantité efficace pour induire l'apoptose de cellules transformées chez un sujet humain ou animal.

**16.** Composition pharmaceutique selon la revendication 14, **caractérisée par le fait qu'**elle contient le composé actif en une quantité efficace pour lever l'inhibition d'un caractère résistant à l'induction d'apoptose de cellules transformées, ce caractère étant dû au gène bcl2, ou au gène MDR, ou au gène BCL-$X_L$, ou à l'enzyme ALDH présent dans ces cellules.

**17.** Composition selon l'une quelconque des revendications 14 à 16, **caractérisée en ce que** la concentration du ou des composé(s) est comprise entre 0,0001 % et 10 % en poids par rapport au poids total de la composition.

**18.** Composition selon l'une quelconque des revendications 14 à 17, **caractérisée en ce qu'**elle comprend en outre au moins un composé choisi parmi les 3-méthylthiopropanoylthio-esters de l'acide thioctique.

**19.** Composition selon la revendication 18, **caractérisée en ce qu'**elle comprend l'iodure de 6-S, 8-S-bis (3-methylthiopropanoyl)octanoate de (2'-trimethylammonium éthyle).

**20.** Composition selon l'une quelconque des revendications 14 à 19 **caractérisée en ce qu'**elle comprend au moins le 4-diméthylamino-4-méthyle-pent-2-ynethioate de S-méthyle et le 4-méthyl-4-morpholin-4-yl-pent-2-ynethioate de S-méthyle.

**21.** Utilisation, pour la préparation d'une composition destinée à induire l'apoptose de cellules transformées, d'au moins un composé selon l'une quelconque des revendications 1 à 10.

**22.** Utilisation, pour la préparation d'une composition destinée à lever l'inhibition d'un caractère résistant à l'induction d'apoptose de cellules transformées, ce caractère étant dû au gène bcl2, ou au gène MDR, ou au gène BCL-$X_L$, ou à l'enzyme ALDH présent dans ces cellules, d'au moins un composé selon l'une quelconque des revendications

1 à 10.

**23.** Utilisation, pour la préparation d'une composition pharmaceutique destinée à lever l'inhibition du caractère résistant à la chimiothérapie, à la radiothérapie ou aux antiandrogènes de cellules transformées, d'au moins un composé selon l'une quelconque des revendications 1 à 10.

**24.** Utilisation selon la revendication 23, **caractérisée en ce que** le caractère résistant à la chimiothérapie, à la radiothérapie ou aux antiandrogènes de cellules transformées est dû au gène bcl2 présent dans ces cellules.

**25.** Utilisation selon la revendication 23, **caractérisée en ce que** le caractère résistant à la chimiothérapie ou aux antiandrogènes de cellules transformées est dû au gène MDR présent dans ces cellules.

**26.** Utilisation selon la revendication 23, **caractérisée en ce que** le caractère résistant à la chimiothérapie ou aux antiandrogènes de cellules transformées est dû au gène BCL-$X_L$ présent dans ces cellules.

**27.** Utilisation selon la revendication 23, **caractérisée en ce que** le caractère résistant à la chimiothérapie, ou aux antiandrogènes de cellules transformées est dû à l'activité de l'enzyme l'ALDH.

**28.** Utilisation d'un composé selon l'une quelconque des revendications 1 à 10, pris seul ou en mélange avec un composé choisi parmi les 3-méthylthiopropanoylthio-esters de l'acide thioctique, pour la préparation d'une composition pharmaceutique destinée au traitement, à la régression et/ou à la prévention des tumeurs cancéreuses.

**29.** Utilisation selon la revendication 28, **caractérisée en ce que** le 3-méthylthiopropanoylthio-ester de l'acide thioctique est l'iodure de 6-S, 8-S-bis (3-methylthiopropanoyl)octanoate de (2'-trimethylammonium éthyle).

**30.** Procédé de traitement cosmétique consistant à appliquer sur la peau ou le cuir chevelu une composition selon l'une quelconque des revendications 14 à 20.

**31.** Procédé selon la revendication 30, **caractérisé en ce qu'**il est destiné au nettoyage de la peau et plus particulièrement des peaux à tendance acnéique ou du cuir chevelu, au traitement ou à la prévention des vergetures, à la protection contre les effets néfastes du soleil, à la prévention et/ou la lutte contre le vieillissement photo-induit ou chronologique ou pour lutter contre l'aspect gras de la peau ou des cheveux.

## Patentansprüche

**1.** Verbindung, **dadurch gekennzeichnet, dass** sie der folgenden allgemeinen Formel (I) entspricht

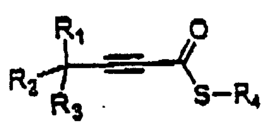

(I)

(I),

worin bedeuten:

- $R_1$:

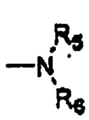 oder 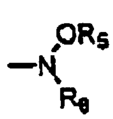 oder 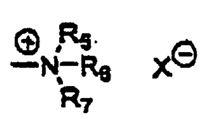 oder 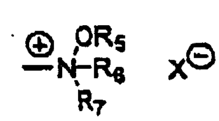

- $R_2$ und $R_3$ unabhängig voneinander eine gesättigte oder ungesättigte, cyclische, geradkettige oder verzweigte Alkylgruppe mit 1 bis 7 Kohlenstoffatomen, eine Arylgruppe mit 1 bis 7 Kohlenstoffatomen oder eine Aralkylgruppe mit 1 bis 12 Kohlenstoffatomen oder die Gruppen $R_2$ und $R_3$ bilden gemeinsam einen gesättigten Alkylring mit 3 bis 7 Kohlenstoffatomen,

- $R_4$ eine gesättigte oder ungesättigte, cyclische, geradkettige oder verzweigte Alkylgruppe mit 1 bis 7 Kohlenstoffatomen, eine Arylgruppe mit 1 bis 7 Kohlenstoffatomen oder eine Aralkylgruppe mit 1 bis 12 Kohlenstoffatomen,
- $R_5$ und $R_6$ unabhängig voneinander eine gesättigte oder ungesättigte, cyclische, geradkettige oder verzweigte Alkylgruppe mit 1 bis 7 Kohlenstoffatomen, eine Arylgruppe mit 1 bis 7 Kohlenstoffatomen oder eine Aralkylgruppe mit 1 bis 12 Kohlenstoffatomen oder die Gruppen $R_5$ und $R_6$ bilden gemeinsam mit dem Stickstoffatom einen gesättigten oder ungesättigten Stickstoffheterocyclus mit 2 bis 6 Kohlenstoffatomen, der gegebenenfalls mit einem Sauerstoffatom, Schwefelatom oder Stickstoffatom substituiert ist, das gegebenenfalls mit einer gesättigten oder ungesättigten, cyclischen, geradkettigen oder verzweigten Alkylgruppe mit 1 bis 7 Kohlenstoffatomen, einer Arylgruppe mit 1 bis 7 Kohlenstoffatomen oder einer Aralkylgruppe mit 1 bis 12 Kohlenstoffatomen substituiert ist,
- $R_7$ ein Wasserstoffatom, eine gesättigte oder ungesättigte, cyclische, geradkettige oder verzweigte Alkylgruppe mit 1 bis 7 Kohlenstoffatomen oder eine Arylgruppe mit 1 bis 7 Kohlenstoffatomen oder eine Aralkylgruppe mit 1 bis 12 Kohlenstoffatomen, und
- X ein Halogenidion oder Nitrat, Sulfat, Sulfonat, Carboxylat, Thiocyanat oder Phosphat.

2. Verbindung nach Anspruch 1, **dadurch gekennzeichnet, dass** die cyclische Alkylgruppe mit 1 bis 7 Kohlenstoffatomen unter Cyclopropyl, Cyclopentyl, Cyclohexyl oder Cycloheptyl ausgewählt ist.

3. Verbindung nach einem der vorhergehenden Ansprüche, **dadurch gekennzeichnet, dass** die geradkettige oder verzweigte gesättigte Alkylgruppe unter Methyl, Ethyl, *n*-Propyl, *i*-Propyl, *n*-Butyl, 2-Butyl, *t*-Butyl, *n*-Pentyl, 2-Pentyl, *i*-Pentyl, *n*-Hexyl, 2-Hexyl, 3-Hexyl, *n*-Heptyl, 2-Heptyl, 3-Heptyl oder *i*-Heptyl ausgewählt ist.

4. Verbindung nach einem der vorhergehenden Ansprüche, **dadurch gekennzeichnet, dass** die Arylgruppe unter Phenyl, Tolyl, Chlorphenyl, Nitrophenyl, Methoxyphenyl, Thiophen oder Furan ausgewählt ist.

5. Verbindung nach einem der vorhergehenden Ansprüche, **dadurch gekennzeichnet, dass** der stickstoffhaltige Heterocyclus unter Pyrrolidin, Piperidin, Morpholin, Thiamorpholin, Piperazin, Homopiperazin oder N-Methylpiperazin ausgewählt ist.

6. Verbindung nach einem der vorhergehenden Ansprüche, **dadurch gekennzeichnet, dass** die ungesättigte Alkylgruppe mit 1 bis 7 Kohlenstoffatomen unter Allyl oder Vinyl ausgewählt ist.

7. Verbindung nach einem der vorhergehenden Ansprüche, **dadurch gekennzeichnet, dass** die Aralkylgruppe mit 1 bis 12 Kohlenstoffatomen unter Benzyl, wobei die Benzylgruppe gegebenenfalls mit einer Alkylgruppe mit 1 bis 5 Kohlenstoffatomen substituiert ist, 1-Phenylethyl, 1-Phenylpropyl, 1-Phenylbutyl und 1-Phenylpentyl ausgewählt ist.

8. Verbindung nach einem der vorhergehenden Ansprüche, **dadurch gekennzeichnet, dass** das Halogenidion unter Fluorid, Chlorid, Bromid oder Iodid ausgewählt ist.

9. Verbindung nach Anspruch 1, **dadurch gekennzeichnet, dass** sie ausgewählt ist unter:

- S-Methyl-4-dimethylamino-4-methyl-pent-2-in-thioat,
- S-Methyl-4-methyl-4-trimethylammonio-pent-2-in-thioatiodid;
- S-methyl-4-di-n-propylamino-4-methyl-pent-2-in-thioat;
- S-Methyl-4-methyl-4-pyrrolidin-1-yl-pent-2-in-thioat und
- S-Methyl-4-methyl-4-morpholin-4-yl-pent-2-in-thioat.

10. Verbindung nach Anspruch 1, **dadurch gekennzeichnet, dass** sie mindestens eine der folgenden Eigenschaften aufweist:

- $R_2$ und $R_3$ bedeuten Methyl;
- $R_4$ bedeutet Methyl;
- $R_5$ und $R_6$ bedeuten unabhängig voneinander eine Alkylgruppe mit einem bis drei Kohlenstoffatomen oder bilden gemeinsam mit dem Stickstoffatom einen Stickstoffheterocyclus, der unter Pyrrolidin, Piperidin oder

Morpholin ausgewählt ist;

- $R_7$ bedeutet Methyl oder Wasserstoff; und
- X bedeutet ein Iodidion, ein Formiation oder ein Carboxylation.

**11.** Verbindung nach einem der Ansprüche 1 bis 10 als Arzneimittel.

**12.** Verwendung einer Verbindung nach einem der Ansprüche 1 bis 10 zur Herstellung eines Arzneimittels, das zur Behandlung der folgenden Erkrankungen vorgesehen ist:

1) kanzerösen oder präkanzerösen Zuständen,
2) dermatologischen Erkrankungen, die mit einer Verhornungsstörung verbunden sind, die auf der Differenzierung und Proliferation beruht, insbesondere zur Behandlung von Acne vulgaris, Acne comedonica, polymorpher Acne, Acne rosaceae, nodulocystischer Acne, Acne conglobata, Acne senilis, sekundären Akneformen, wie Acne solaris, Acne medikamentosa oder Acne professionalis, und/oder
3) Ichtyosis, ichtyosisartigen Zuständen, Palmoplantarkeratosen, Leucoplakien und leucoplakieformen Zuständen, und/ oder
4) dermatologischen Erkrankungen mit entzündlicher immunoallergischer Komponente mit oder ohne Proliferationsstörung der Zellen, insbesondere Psoriasis, Psoriasis arthropathica, Ekzemen, Atopie der Atemwege und Hypertrophie des Zahnfleisches, und/oder
5) Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida, T-Lymphomen und Proliferationen, die von UV-Strahlung induziert werden können, insbesondere im Falle von Epithelioma basocellulare und spinocellulare, sowie präkanzerösen Lesionen der Haut, wie Keratoakanthomen, und/oder
6) Immundermatosen, wie Lupus erythematodes, bullöse Immunerkrankungen und Kollagenerkrankungen, wie Sklerodermie, und/ oder
7) dermatologischen Störungen oder allgemeinen Störungen mit immunologischer Komponente, und/ oder
8) Funktionsstörungen der Talgdrüse, wie Hyperseborrhoe bei Akne oder einfache Seborrhoe, und/oder
9) Hautstörungen, die von einer Exposition gegenüber UV-Strahlung herrühren, lichtinduzierter oder altersbedingter Hautalterung, aktinischen Pigmentierungen und Keratosen oder Pathologien, die mit der altersbedingten oder aktinischen Alterung zusammenhängen, und/ oder
10) Störungen der Wundheilung oder Streifen, und/ oder
11) entzündlichen Erkrankungen, wie Arthritis, und/oder
12) Erkrankungen viraler Herkunft der Haut oder allgemeinen Erkrankungen viraler Herkunft, wie des Kaposi-Syndrom oder von Hepatitis, und/oder
13) ophthalmologischen Störungen, insbesondere Corneopathien.

**13.** Verwendung einer Verbindung nach einem der Ansprüche 1 bis 10 zur Herstellung eines Arzneimittels, das zur Behandlung von kanzerösen oder präkanzerösen Zuständen vorgesehen ist.

**14.** Zusammensetzung, **dadurch gekennzeichnet, dass** sie in einem physiologisch akzeptablen Träger mindestens eine Verbindung nach einem der Ansprüche 1 bis 10 enthält.

**15.** Zusammensetzung nach Anspruch 14, **dadurch gekennzeichnet, dass** sie den Wirkstoff in einer Menge enthält, die wirksam ist, um die Apoptose von transformierten Zellen bei einem Menschen oder einem Tier zu induzieren.

**16.** Pharmazeutische Zusammensetzung nach Anspruch 14, **dadurch gekennzeichnet, dass** sie den Wirkstoff in einer Menge enthält, die wirksam ist, um die Inhibierung einer Resistenzeigenschaft gegenüber der Induktion der Apoptose von transformierten Zellen aufzuheben, wobei diese Eigenschaft von dem Gen BCL2 oder dem Gen MDR oder dem Gen BCL-$X_L$ oder dem Enzym ALDH in den Zellen herrührt.

**17.** Zusammensetzung nach einem der Ansprüche 14 bis 16, **dadurch gekennzeichnet, dass** die Konzentration der Verbindung(en) im Bereich von 0,0001 bis 10 Gew.-%, bezogen auf das Gesamtgewicht der Zusammensetzung, liegt.

**18.** Zusammensetzung nach einem der Ansprüche 14 bis 17, **dadurch gekennzeichnet, dass** sie ferner mindestens eine Verbindung enthält, die unter den 3-Methylthiopropanoylthioestern von Thioctansäure ausgewählt ist.

**19.** Verbindung nach Anspruch 18, **dadurch gekennzeichnet, dass** sie das (2'-Trimethylammonioethyl)-6-S-8-S-bis (3-methylthiopropanoyl)octanoat-iodid enthält.

**20.** Zusammensetzung nach einem der Ansprüche 14 bis 19, **dadurch gekennzeichnet, dass** sie zumindest das S-Methyl-4-dimethylamino-4-methyl-pent-2-in-thioat und das S-Methyl-4-methyl-4-morpholin-4-yl-pent-2-in-thioat enthält.

**21.** Verwendung mindestens einer Verbindung nach einem der Ansprüche 1 bis 10 zur Herstellung einer Zusammensetzung, die dazu vorgesehen ist, die Apoptose von transformierten Zellen zu induzieren.

**22.** Verwendung mindestens einer Verbindung nach einem der Ansprüche 1 bis 10 zur Herstellung einer Zusammensetzung, die dazu vorgesehen ist, die Inhibierung einer Resistenzeigenschaft gegenüber der Induktion der Apoptose von transformierten Zellen aufzuheben, wobei diese Eigenschaft mit dem Gen BCL2, dem Gen MDR, dem Gen BCL-$X_L$ oder dem Enzym ALDH in den Zellen zusammenhängt.

**23.** Verwendung mindestens einer Verbindung nach einem der Ansprüche 1 bis 10 zur Herstellung einer pharmazeutischen Zusammensetzung, die dazu vorgesehen ist, die Inhibierung der Resistenz gegenüber Chemotherapie, Radiotherapie oder Antiandrogenen von transformierten Zellen aufzuheben.

**24.** Verwendung nach Anspruch 23, **dadurch gekennzeichnet, dass** die Resistenz der transformierten Zellen gegenüber Chemotherapie, Radiotherapie oder Antiandrogenen von dem in den Zellen vorliegenden Gen BCL2 verursacht wird.

**25.** Verwendung nach Anspruch 23, **dadurch gekennzeichnet, dass** die Resistenz der transformierten Zellen gegenüber Chemotherapie oder Antiandrogenen von dem in den Zellen vorliegenden Gen MDR verursacht wird.

**26.** Verwendung nach Anspruch 23, **dadurch gekennzeichnet, dass** die Resistenz der transformierten Zellen gegenüber Chemotherapie oder Antiandrogenen von dem in den Zellen vorliegenden Gen BCL-$X_L$ verursacht wird.

**27.** Verwendung nach Anspruch 23, **dadurch gekennzeichnet, dass** die Resistenz der transformierten Zellen gegenüber Chemotherapie oder Antiandrogenen von der Aktivität des Enzyms ALDH verursacht wird.

**28.** Verwendung einer Verbindung nach einem der Ansprüche 1 bis 10 alleine oder im Gemisch mit einer Verbindung, die unter den 3-Methylthiopropanoylthioestern von Thioctansäure ausgewählt ist, zur Herstellung einer pharmazeutischen Zusammensetzung, die zur Behandlung, Regression und/ oder Prävention von Krebstumoren vorgesehen ist.

**29.** Verwendung nach Anspruch 28, **dadurch gekennzeichnet, dass** der 3-Methylthiopropanoylthioester von Thioctansäure das (2'-Trimethylammonioethyl)-6-S-8-S-bis(3-methylthiopropanoyl)-octanoat-iodid ist.

**30.** Verfahren zur kosmetischen Behandlung, das darin besteht, auf die Haut oder die Kopfhaut eine Zusammensetzung nach einem der Ansprüche 14 bis 20 aufzutragen.

**31.** Verfahren nach Anspruch 30, **dadurch gekennzeichnet, dass** es zur Reinigung der Haut und insbesondere von Hauttypen mit Aknetendenz oder der Kopfhaut, zur Behandlung oder Vorbeugung von Streifen, zum Schutz gegen die schädlichen Wirkungen der Sonne, zur Vorbeugung und/oder Bekämpfung der lichtinduzierten oder altersbedingten Alterung oder zur Bekämpfung des fettigen Aussehens der Haut oder der Haare vorgesehen ist.

## Claims

**1.** Compound, **characterized in that** it corresponds to the general formula (I) below:

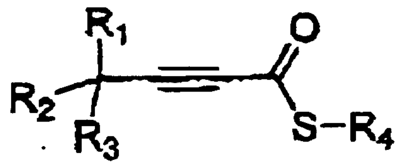

(I)

in which:

- $R_1$ represents:

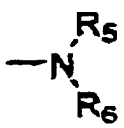 or 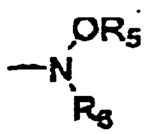 or 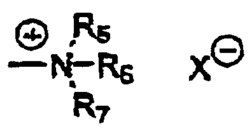 or 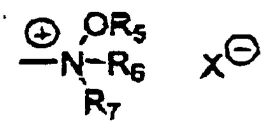

- $R_2$ and $R_3$, independently, represent a saturated or unsaturated, cyclic, linear or branched alkyl radical containing from 1 to 7 carbon atoms or an aryl radical containing from 1 to 7 carbon atoms, or an aralkyl radical containing from 1 to 12 carbon atoms or, taken together, form a saturated alkyl ring containing from 3 to 7 carbon atoms,
- $R_4$ represents a saturated or unsaturated, cyclic, linear or branched alkyl radical containing from 1 to 7 carbon atoms or an aryl radical containing from 1 to 7 carbon atoms or an aralkyl radical containing from 1 to 12 carbon atoms,
- $R_5$ and $R_6$, independently, represent a saturated or unsaturated, cyclic, linear or branched alkyl radical containing from 1 to 7 carbon atoms or an aryl radical containing from 1 to 7 carbon atoms, or an aralkyl radical containing from 1 to 12 carbon atoms or, taken together, form, with the nitrogen atom, a saturated or unsaturated nitrogen heterocycle containing from 2 to 6 carbon atoms, optionally substituted with an oxygen, a sulphur or with a nitrogen atom optionally substituted with a saturated or unsaturated, cyclic, linear or branched alkyl radical containing from 1 to 7 carbon atoms or an aryl radical containing from 1 to 7 carbon atoms or an aralkyl radical containing from 1 to 12 carbon atoms,
- $R_7$ represents a hydrogen atom, a saturated or unsaturated, cyclic, linear or branched alkyl radical containing from 1 to 7 carbon atoms or an aryl radical containing from 1 to 7 carbon atoms or an aralkyl radical containing from 1 to 12 carbon atoms, and
- X represents a halide ion or a nitrate, sulphate, sulphonate, carboxylate, thiocyanate or phosphate anion.

**2.** Compound according to Claim 1, **characterized in that** the cyclic alkyl radical containing from 1 to 7 carbon atoms is chosen from the cyclopropyl, cyclopentyl, cyclohexyl or cycloheptyl radical.

**3.** Compound according to either of the preceding claims, **characterized in that** the linear or branched saturated alkyl radical is chosen from the methyl, ethyl, n-propyl, i-propyl, n-butyl, 2-butyl, t-butyl, n-pentyl, 2-pentyl, i-pentyl, n-hexyl, 2-hexyl, 3-hexyl, n-heptyl, 2-heptyl, 3-heptyl or i-heptyl radical.

**4.** Compound according to any one of the preceding claims, **characterized in that** the aryl radical is chosen from the phenyl, tolyl, chlorophenyl, nitrophenyl, methoxyphenyl, thiophene or furan radical.

**5.** Compound according to any one of the preceding claims, **characterized in that** the nitrogen heterocycle radical is chosen from pyrrolidine, piperidine, morpholine, thiamorpholine, piperazine, homopiperazine and N-methylpiperazine.

**6.** Compound according to any one of the preceding claims, **characterized in that** the unsaturated alkyl radical containing from 1 to 7 carbon atoms is chosen from the allyl or vinyl radical.

**7.** Compound according to any one of the preceding claims, **characterized in that** the aralkyl radical containing from 1 to 12 carbon atoms is chosen from the benzyl radical optionally substituted with an alkyl radical containing from

1 to 5 carbon atoms, and 1-phenylethyl, 1-phenylpropyl, 1-phenylbutyl and 1-phenylpentyl radicals.

**8.** Compound according to any one of the preceding claims, **characterized in that** the halide ion is chosen from the fluoride, chloride, bromide and iodide.

**9.** Compound according to Claim 1, **characterized in that** it is chosen from:

- S-methyl 4-dimethylamino-4-methyl-2-pentynethioate;
- S-methyl 4-methyl-4-trimethylammonium-2-pentynethioate iodide;
- S-methyl 4-di-n-propylamino-4-methyl-2-pentynethioate;
- S-methyl 4-methyl-4-pyrrodin-1-yl-2-pentynethioate;
- and S-methyl 4-methyl-4-morpholin-4-yl-2-pentynethioate.

**10.** Compound according to Claim 1, **characterized in that** it has at least one of the following characteristics:

- $R_2$ and $R_3$ represent a methyl radical;
- $R_4$ represents a methyl radical;
- $R_5$ and $R_6$, independently, represent an alkyl radical containing from one to three carbon atoms or, taken together, form, with the nitrogen atom, a nitrogen heterocycle chosen from pyrrolidine, piperidine and morpholine;
- $R_7$ represents a methyl radical or a hydrogen atom; and
- X represents an iodide ion, a formate ion or a carboxylate ion.

**11.** Compound according to any one of Claims 1 to 10, as medicinal product.

**12.** Use of a compound according to any one of Claims 1 to 10, for the manufacture of a medicinal product intended for the treatment of:

1) cancerous or precancerous conditions,
2) dermatological complaints associated with a keratinization disorder relating to differentiation and to proliferation, in particular common acne, comedones, polymorphs, rosacea, nodulocystic acne, acne conglobata, senile acne and secondary acne such as solar, medicinal or occupational acne, and/or
3) ichthyosis, ichthyosiform conditions, palmoplantar keratoderma, leukoplakia and leukoplakiform conditions, and/or
4) dermatological complaints with an inflammatory immuno-allergic component, with or without a cellular proliferation disorder, and in particular psoriasis, arthropathia psoriatica, eczema, respiratory atopy and gingival hypertrophy, and/or
5) dermal or epidermal proliferations, whether benign or malignant, whether or not of viral origin, such as common warts, flat warts and epidermodysplasia verruciformis, oral or florid papillomatoses, T lymphoma and proliferations which may be induced by ultraviolet light, in particular in the case of basal cell and spinocellular epithelioma, and also precancerous skin lesions such as keratoacanthomas, and/or
6) immune dermatitides such as lupus erythematosus, bullous immune diseases and collagen diseases, such as scleroderma, and/or
7) dermatological or systemic complaints with an immunological component, and/or
8) sebaceous function disorders such as the hyperseborrhoea of acne or simple seborrhoea, and/or
9) skin disorders due to exposure to UV radiation, light-induced or chronological ageing of the skin, or pigmentations and actinic keratoses, or pathologies associated with chronological or actinic ageing, and/or
10) cicatrization disorders or stretch marks, and/or
11) inflammatory complaints such as arthritis, and/or
12) cutaneous or systemic complaints of viral origin, such as Kaposi's syndrome or hepatitis, and/or
13) ophthalmological disorders, in particular corneopathies.

**13.** Use of a compound according to any one of Claims 1 to 10, for the manufacture of a medicinal product intended for the treatment of cancerous or precancerous conditions.

**14.** Composition, **characterized in that** it comprises, in a physiologically acceptable support, at least one of the compounds according to any one of Claims 1 to 10.

**15.** Composition according to Claim 14, **characterized in that** it contains the active compound in an amount which is effective to induce apoptosis of transformed cells in a human or animal individual.

**16.** Pharmaceutical composition according to Claim 14, **characterized in that** it contains the active compound in an amount which is effective to remove the inhibition of the resistant nature to the induction of apoptosis of transformed cells, this nature being due to the $bcl_2$ gene or to the MDR gene or to the $BCL\text{-}X_L$ gene, or to the enzyme ALDH present in these cells.

**17.** Composition according to any one of Claims 14 to 16, **characterized in that** the concentration of the compound (s) is between 0.0001% and 10% by weight relative to the total weight of the composition.

**18.** Composition according to any one of Claims 14 to 17, **characterized in that** it also comprises at least one compound chosen from 3-methylthiopropanoyl thioesters of thioctic acid.

**19.** Composition according to Claim 18, **characterized in that** it comprises 2'-(trimethylammonium)ethyl 6S,8S-bis (3-methylthiopropanoyl)octanoate iodide.

**20.** Composition according to any one of Claims 14 to 19, **characterized in that** it comprises at least S-methyl 4-dimethylamino-4-methyl-2-pentynethioate and S-methyl 4-methyl-4-morpholin-4-yl-2-pentynethioate.

**21.** Use, for the preparation of a composition intended to induce apoptosis of transformed cells, of at least one compound according to any one of Claims 1 to 10.

**22.** Use, for the preparation of a composition intended to remove the inhibition of the resistant nature to the induction of apoptosis of transformed cells, this nature being due to the $bcl_2$ gene or the MDR gene or to the $BCL\text{-}X_L$ gene, or to the enzyme ALDH present in these cells, of at least one compound according to any one of Claims 1 to 10.

**23.** Use, for the preparation of a pharmaceutical composition intended to remove the inhibition of the chemotherapy-resistant, radiotherapy-resistant or antiandrogen-resistant nature of transformed cells, of at least one compound according to any one of Claims 1 to 10.

**24.** Use according to Claim 23, **characterized in that** the chemotherapy-resistant, radiotherapy-resistant or antiandrogen-resistant nature of transformed cells is due to the $bcl_2$ gene present in these cells.

**25.** Use according to Claim 23, **characterized in that** the chemotherapy-resistant or antiandrogen-resistant nature of transformed cells is due to the MDR gene present in these cells.

**26.** Use according to Claim 23, **characterized in that** the chemotherapy-resistant or antiandrogen-resistant nature of transformed cells is due to the $BCL\text{-}X_L$ gene present in these cells.

**27.** Use according to Claim 23, **characterized in that** the chemotherapy-resistant or antiandrogen-resistant nature of transformed cells is due to the activity of the enzyme ALDH.

**28.** Use of a compound according to any one of Claims 1 to 10, taken alone or as a mixture with a compound chosen from 3-methylthiopropanoyl thioesters of thioctic acid, for the preparation of a pharmaceutical composition intended for the treatment, regression and/or prevention of cancer tumours.

**29.** Use according to Claim 28, **characterized in that** the 3-methylthiopropanoyl thioester of thioctic acid is 2'-(trimethylammonium)ethyl 6S,8S-bis(3-methylthiopropanoyl)octanoate iodide.

**30.** Cosmetic treatment process which consists in applying a composition according to any one of Claims 14 to 20 to the skin or the scalp.

**31.** Process according to Claim 30, **characterized in that** it is intended for cleansing the skin, and more particularly skin with a tendency towards acne, or the scalp, for treating or preventing stretch marks, for protecting against the harmful effects of sunlight, for preventing and/or combating light-induced or chronological ageing or for combating the greasy appearance of the skin or the hair.

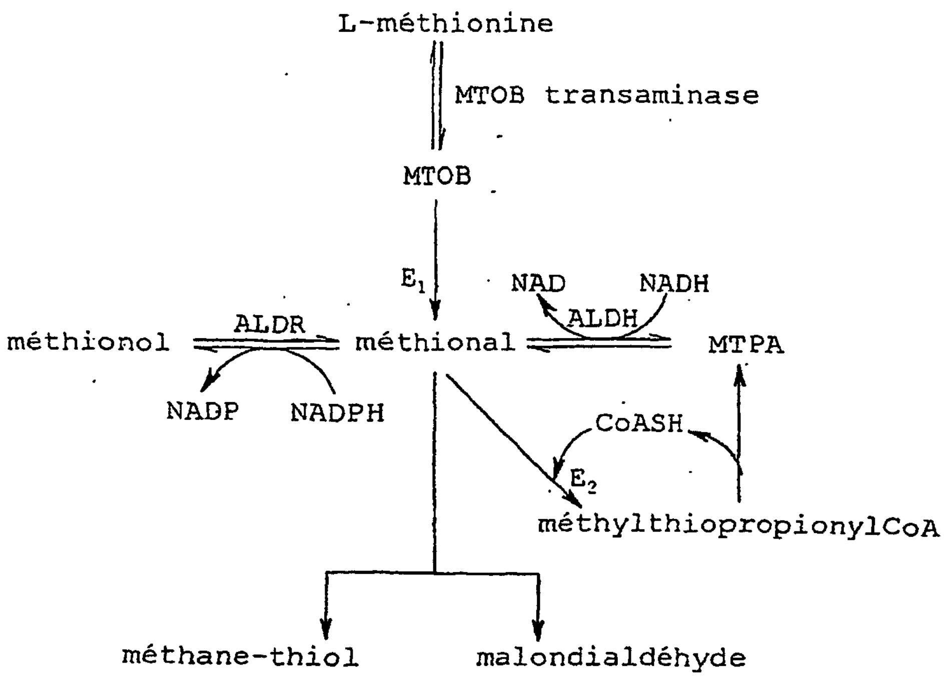


FIGURE 1

- MTOB représente l'acide 4-méthylthio-2-oxobutanoïque,
- MTPA représente l'acide méthylthiopropionique,
- $E_1$ représente la décarboxylase du complexe déshydrogénase oxo-acide à chaîne branchée dont le co-facteur est la thiamine pyrophosphate,
- $E_2$ représente la transacylase du complexe déshydrogénase oxo-acide à chaîne branchée dont le co-facteur est l'acide thioctique,
- ALDR représente l'aldéhyde réductase,
- ALDH représente l'aldéhyde déshydrogénase,
- CoASH représente l'acétyl coenzyme A,
- NADH/NAD représente le nicotinamide adénine dinucléotide, et
- NADPH/NADP représente le nicotinamide adénine dinucléotide phosphate.

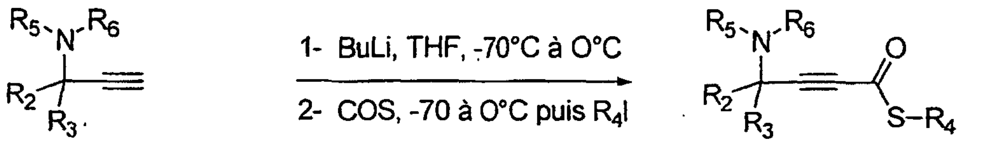


(III)

(I) $R_1$ =N($R_5R_6$)

figure -2

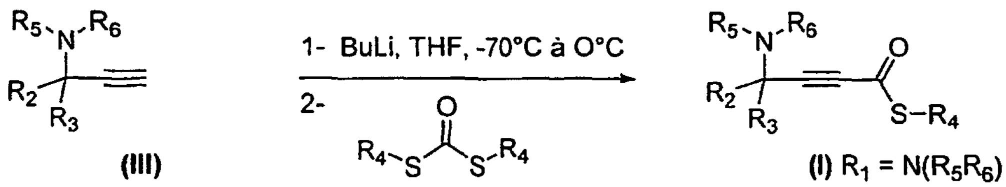


(III)

(IV)

(I) $R_1$ = N($R_5R_6$)

figure -3

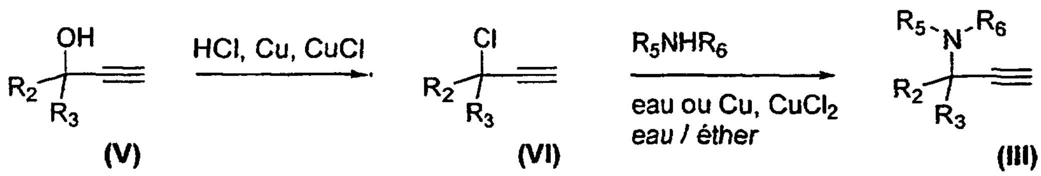


(V)

(VI)

(III)

figure -4

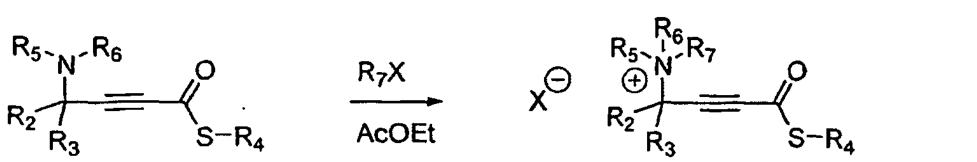


(I) $R_1$ = N($R_5R_6$)

(I) $R_1$ = $N^+$($R_5R_6R_7$), $X^-$

figure -5

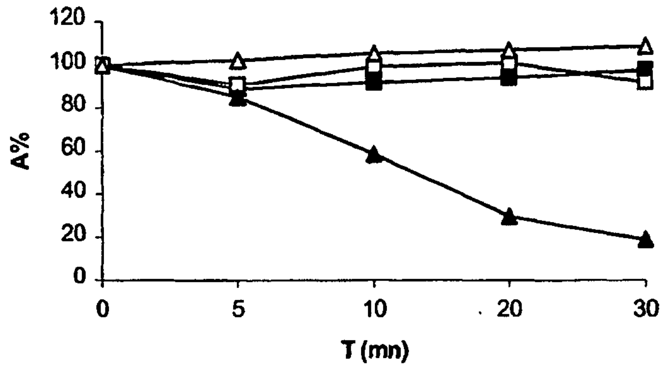


Figure -6

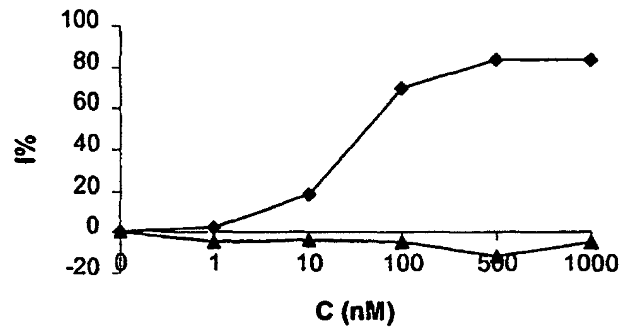


Figure -7

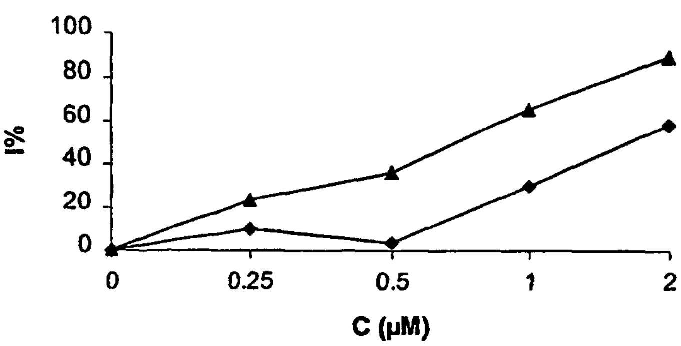


Figure -8